# EUROPEAN PATENT SPECIFICATION

(11) **EP 1 572 014 B1**
(45) Date of publication and mention of the grant of the patent: **25.07.2007**
(21) Application number: 03780558.7
(22) Date of filing: 16.12.2003
(51) Int. Cl.: A61B 17/34, A61B 17/02

(54) **A SURGICAL DEVICE**
CHIRURGISCHE VORRICHTUNG
DISPOSITIF CHIRURGICAL

(30) Priority: 16.12.2002 US 433603 P; 11.03.2003 US 453200 P
(43) Date of publication of application: 14.09.2005
(73) Proprietor: Atropos Limited, Bray County Wicklow (IE)
(72) Inventor: BONADIO, Frank, Bray, County Dublin (IE); BUTLER, John, Deansgrange, County Dublin (IE); VAUGH, Trevor, Birr, County Offaly (IE); MACNALLY, Shane Joseph, Bray, County Wicklow (IE); REID, Alan, Clontarf, Dublin 3 (IE)
(74) Representative: Roche, Dermot
(86) International application number: PCT/IE2003/000171
(87) International publication number: WO 2004/054456

(56) References cited:
- EP-A- 0 950 376
- WO-A-95/07056
- US-A- 5 480 410
- US-A- 5 906 577
- US-A1- 2001 037 053

## Description

The invention relates to a medical device particularly for use in surgery to provide surgical access to the abdomen and maintain a substantially gas-tight seal around the arm or an instrument during surgery. Surgery of this type is referred to as hand-assisted laparoscopic surgery or hand-access surgery.

Conventional abdominal surgery requires the creation of an incision in the abdominal wall to allow access to, and visualisation of the internal organs and other anatomical structures. These incisions must be large enough to accommodate the surgeon's hands and any instruments to be utilised by the surgeon during the surgery. Traditionally the size of these incisions has been dictated by the need to see, retract and palpate internal bodily structures. While a large incision will provide access to the interior of the abdomen they are associated with longer healing times, are more susceptible to infection and result in unsightly scars.

Alternatives to open surgery exist in the form of endoscopic or laparoscopic surgery. In this method of surgery, the surgeon operates through small incisions using remotely actuated instruments. The instruments pass through the abdominal wall using devices called trocars. These working channels typically have a diameter ranging from 5 to 25 millimetres. Vision is provided using a laparoscope which is typically 20 to 25 centimetres long and uses fibre-optic technology or a CCD camera to provide the operator with a picture of the interior of the abdomen. The abdomen must be insufflated with a gas such as carbon dioxide or nitrogen to maintain a bubble effect and provide a viable working space for the operator to perform the surgery unhindered by the lack of space. This insufflation creates a working space known as the pneumoperitoneum. Trocars through which instruments are inserted are constructed to prevent loss of the gas through them resulting in collapse of the pneumoperitoneum.

The benefits of laparoscopic surgery are numerous. Recovery times have been shown to be reduced due to the absence of a large incision. This has benefits for the patient, the health care organisation and society. The benefits to the patient are reduced stay in hospital, faster mobilisation and return to normal activity. The benefits to the health care organisation is also due to the reduced stay in hospital which is often the most expensive aspect of health care provision. Society benefits in faster return to work and normal activity of the patient.

However, not all surgical procedures can be performed laparoscopically. Surgery requiring the removal of large organ specimens, such as surgery for removal of the colon, has traditionally been hampered by the small incisions used for the introduction of laparoscopic instruments in the surgery.

The other major disadvantages of laparoscopic surgery are due to the complex nature of the technique. Surgeons who wish to practise laparoscopic surgery must spend much time training to master the technique. The success of laparoscopic surgery depends on the skill of the surgeon to manipulate organs and carry out delicate tasks using remotely actuated instruments. Unfortunately in laparoscopic surgery the surgeon is insulated from the material that they are working on. This deprives the surgeon of tactile feedback and the ability to palpate delicate structures. The surgeon's most effective instrument, the hand, is reduced to a device that must simply actuate instruments that are inherently lacking in dexterity and operability due to the constraints on their design placed by the nature of the narrow channels in trocars through which they must pass. Another disadvantage of laparoscopy is that the image viewed by the surgeon is a two dimensional image on a video screen. The surgeon loses three dimensional perspective of depth and distance and awareness of the proximity of other structures during video laparoscopy.

These disadvantages have led to long learning curves for the practitioners of laparoscopic surgery, required highly skilled and co-ordinated surgical teams and has limited the application of laparoscopic surgery to relatively simple surgical procedures.

Recently, new surgical techniques have been developed that combine the advantages of both open surgery and laparoscopic surgery. In these new techniques surgery is carried out using a laparoscopic approach with the addition of a slightly larger incision to allow the surgeon to insert a hand into the insufflated abdomen. This is often referred to as hand-assisted laparoscopic surgery or HALS.

HALS allows surgeons to regain the tactile feedback and three-dimensional perspective lost in the conversion from open to laparoscopic procedures. It also permits rapid finger dissection, enhanced retraction capabilities and simplified haemostasis. There are several publications in the literature describing procedures carried out using a hand-assisted approach. These include total and sub-total colectomy, rectopexy, Nissen's fundoplication, gastrectomy, splenectomy, nephrectomy, pancreatectomy and others. Some of these procedures were previously performed using an open technique only. Over the past few years several centres have been investigating HALS with surgical device companies and increasing the literature on the subject. With the advent of surgical devices for facilitating HALS it is expected that more open surgical procedures will be converted to HALS procedures.

It is important that devices for use in hand-assisted laproscopic surgery provide a seal to a surgeon's arm to maintain the pneumoperitoneum required.

US patent application published under No. US 2001/0037053 discloses a wound protector retractor comprising an outer mounting assembly and a connecting sleeve. The outer mounting assembly is provided by rings which maybe rotated to form a central lumen of reduced cross-section.

US patent No. 5,480,410 discloses a medical device including one or more iris valves.

Various hand access devices have been developed; however, there is a need for an improved hand access device which will be easy to use.

### Statements of Invention

According to the invention there is provided a surgical device for use in surgery comprising
a mounting element;
a sealing member of pliable material mounted to the mounting element, the sealing member having a normally closed access opening; and
at least a portion of the sealing member being movable on insertion of an object such as a surgeon's hand to open the access opening whilst maintaining substantial sealing engagement with the object; and
a spring element to bias the sealing member to close the access opening.

In one embodiment the mounting element comprises a first ring element and a second ring element and the sealing member extends between the ring elements.

In one embodiment the device comprises a biasing means to bias the sealing member to close the access opening.

The biasing means may be provided by pre-tensioning the sealing member to close the access opening.

In one embodiment the sealing member comprises a sleeve. The sleeve may be twisted to define the normally closed access opening.

In one embodiment the sealing member is of an elastomeric material.

The spring element may extend between the first
and second ring elements. The spring element may have opposite ends and at least one of the ends is attached to a ring element.

In one embodiment one end of the spring is attached to the first ring element and the other end of the spring is attached to the second ring element. The first ring element may be a fixed ring element and a second ring element is rotatable relative to the first ring element.

In one embodiment one ring element is located within the other ring element.

One ring element may have a rib which is locatable in a corresponding groove in the other ring element.

In one embodiment the sealing member is a sleeve, one end of the sleeve being mounted to one ring element and an opposite end of the sleeve being mounted to the other ring element. The sealing member may be anchored to both the first and second ring elements.

In one embodiment the sleeve has a first mounting ring at one end for mounting to the first ring element and a second mounting ring at an opposite end for mounting to the second ring element.

In one embodiment the mounting ring elements have locators for a biassing element extending between the ring elements. The locator may comprise a groove.

In one embodiment the sealing member comprises a sleeve having an untwisted configuration and a twisted sealing configuration, the length of the sleeve in the untwisted configuration which is less than a diameter of the sleeve.

One ring may be rotatable relative to the other ring element.

In one embodiment the mounting element has an attachment for mounting to another device such as a retractor. The attachment may comprise a groove.

In one case the other device comprises a sleeve element and the attachment is engagable with the sleeve element.

The mounting element may be movable relative to the other device. In one case the mounting element is slidable relative to the other device.

In one embodiment the other device comprises a sealing device. The sealing device may be located proximally of the sealing member.

In one embodiment the sealing device comprises a lip seal.

The invention also provides a hand access device for use in hand assisted laparoscopic surgery comprising a device of the invention.

The invention further provides a surgical device assembly comprising a retractor and a device of the invention. The device may be releasably mountable to the retractor. Alternatively the device is mounted to the retractor.

Herein there is also disclosed a method of performing hand assisted surgery comprising the steps of:-
providing a device of the invention;
inserting a hand into the device against the biasing of the sealing member whilst maintaining substantial sealing engagement between the sealing member and the surgeon's hand/arm.

Herein there is further disclosed a method of performing a surgical procedure comprising the steps of providing a device of the invention and inserting an object into the device against the biasing of the sealing member whilst maintaining sealing engagement between the sealing member and the object. The object may be surgeon's arm/forearm or an instrument.

The retractor member may comprise a distal portion for insertion through an incision made in a patient, and a proximal portion for extending from the incision and outside of the patient;
a distal member associated with the distal portion of the retractor member;
a proximal member associated with the proximal portion of the retractor member;
the retractor member being axially movable relative to the distal member to draw the proximal and distal members towards one another thereby shortening the axial extent of the retractor member between the proximal and distal members.

In one embodiment the retractor member comprises a sleeve member. The sleeve member preferably extends around the distal member.

In one embodiment the distal member is a ring member such as a resilient ring member, for example, an O-ring.

In one embodiment the proximal member is connected to the retractor member. The proximal member may be a ring member.

In one embodiment the sleeve member is of a pliable material.

In one arrangement the sleeve extends from the proximal member, around the distal member and has a return section outside of the proximal member.

The return section may have a handle member such as a ring member.

In one embodiment the device comprises a guide member.

The retractor member may extend between the guide member and the proximal member.

The guide member may comprise a receiver for the proximal member.

The guide member may comprise a guide ring-receiving member.

The sleeve return section may be configured to provide an integral valve member. In this case the sleeve return section may be twisted to provide an iris valve.

In another embodiment the sleeve return section is mounted to the guide member.

The sleeve return section may be extended into the opening defined by the sleeve member.

The device may comprise a lock for locking the guide member to the proximal member. Typically the guide member is engagable with the proximal member to provide the lock.

The guide member may be an interference fit with the proximal member.

In one embodiment of the invention the device includes a valve, such as an iris-type valve.

In one embodiment the device comprises a biasing member for biasing the valve into a desired position such as the closed position.

In one arrangement the device comprises a guide member located proximally of the proximal member and a biasing means is provided between the proximal member and the guide member. The biasing means may comprise a spring such as a coil spring.

In one embodiment a sleeve member extends between the proximal member and the guide member and the biasing means is located around the sleeve. The sleeve member may be an extension of the retractor member.

In one embodiment the device comprises a release member for releasing the device from an incision. The release member may comprise an elongate member such as a pull ribbon or string extending from a distal end of the device.

The release member may extend from the distal member.

In one embodiment the valve is located or locatable proximal of the proximal member. A pliable material may be provided between the valve and the proximal member. The pliable material may comprise a proximal extension of the retractor member.

In one embodiment the pliable material comprises a sleeve section.

In another embodiment the valve is a lip seal.

Herein there is also disclosed a method which includes retracting an incision by carrying out the steps of:-
providing a device comprising a retractor member having a distal portion and a proximal portion, a distal member associated with the distal portion and a proximal portion associated with the proximal portion;
inserting the distal member and the distal portion of the retractor member through an incision made in a patient; and
pulling the retractor member axially relative to the distal member to draw the proximal and distal members towards one another thereby shortening the axial extent of the retractor member between the proximal and distal members and retracting the incision.

### Brief Description of the Drawings

The invention will be more clearly understood from the following description of some embodiments thereof, given by way of example only, with reference to the accompanying drawings, in which:-
Fig. 1 is a perspective view of a retractor;
Fig. 2 is a cross sectional view of the device of Fig. 1;
Figs. 3 and 4 are perspective views illustrating the formation of the device of Figs. 1 and 2;
Figs. 5 and 6 are cross sectional views of Figs. 3 and 4 respectively;
Figs. 7 and 8 are perspective views illustrating the use of the device;
Figs. 9 and 10 are cross sectional views illustrating the method of use of the device;
Fig. 11 is a cross sectional view of another retractor device in a configuration ready for use;
Fig. 12 is a perspective view of the device of Fig. 11 with a distal portion inserted through an incision;
Fig. 13 is a cross sectional view of the device of Fig. 11 with a distal portion inserted through an incision;
Fig. 14 is a cross sectional view of the device of Fig. 11 in use with an incision retracted;
Fig. 15 is a perspective view of the device in the configuration of Fig. 14;
Fig. 16 is a perspective view of the device in situ with an excess sleeve portion being removed;
Fig. 17 is a cross sectional view of the device in situ with an excess sleeve portion extending back into the incision;
Fig. 18 is a perspective view of the device in situ with a excess sleeve portion being twisted;
Fig. 19 is a perspective view similar to Fig. 18 with the excess sleeve portion further twisted to provide an iris valve;
Fig. 20 is a cross sectional view of another device in situ;
Fig. 21 is a cross sectional view of the device of Fig. 20 with an excess sleeve portion mounted to a guide member;
Fig. 22 is a cross sectional view of the device of Fig. 21 with the excess sleeve portion inflated to provide an integral everting access part;
Fig. 23 is a perspective view of another retractor incorporating a release device;
Fig. 24 is a cross sectional view of the retractor of Fig. 23;
Fig. 25 is a perspective view illustrating the formation of the device of Fig. 23;
Fig. 26 is a cross sectional view of the device in the configuration of Fig. 25;
Fig. 27 is a cross sectional view of the retractor of Figs. 23 to 26, in use;
Fig. 28 is a cross sectional view of the retractor of Figs. 23 to 27 illustrating the operation of a release device;
Fig. 29 is a perspective view of another device in an insertion configuration;
Fig. 30 is a perspective view of the device of Fig. 29 in position in an incision;
Fig. 31 is another perspective view of the device of Fig. 30 in another configuration;
Fig. 32 is another view of the device of Fig 31 with an outer portion severed and a valve being formed;
Fig. 33 is a view of the device of Fig. 32 with the valve closed;
Fig. 34 is a perspective view of another device similar to the device of Figs. 29 to 33 with a valve closed;
Fig. 35 is a cross sectional view of the device of Fig. 34;
Fig. 36 is a perspective view of another device similar to the device of Figs. 29 to 33 incorporating a biasing means in an inserted configuration;
Fig. 37 is another perspective view of the device of Fig. 36 in a retracting configuration;
Fig. 38 is a perspective view of the device of Fig. 37 in another configuration and excess sleeve being removed;
Fig. 39 is a perspective view of the device of Fig. 38 with a valve closed;
Fig. 40 is a perspective view of the device of Fig. 39 with a valve partially open;
Fig. 41 is a perspective view of the device of Fig. 39 with an object inserted through the valve;
Fig. 42 is a perspective view of another device;
Fig. 43 is a cross sectional view of the device of Fig. 42 in position in an incision;
Fig. 44 is a cross sectional view of the device of Fig. 43 with an object extending therethrough;
Fig. 45 is a cross sectional view similar to Fig. 44 with an object offset from a longitudinal axis of the device;
Fig. 46 is a cross sectional view of another device on insertion into an incision;
Fig. 47 is a cross sectional view of the device of Fig. 46 with an incision retracted;
Fig. 48 and 49 are cross sectional views of the device of Fig. 47 showing the formation of an iris valve;
Fig. 49(a) is a cross sectional view of another device;
Fig. 49(b) is a plan view of a hand access device of the invention in a closed position;
Fig. 49(c) is a plan view of the device of Fig. 49(b) in an opened position;
Fig. 49 (d) is a plan view showing the opening of the device of Figs. 49 (b) and 49 (c);
Figs. 49(e) and (f) are, respectively, plan and side views of the hand access device of Fig. 49(b) in a closed position;
Figs. 49(g) and (h) are views similar to Fig. 49(e) and (f) with the device in an open position;
Fig. 49(i) is a cross sectional view of a hand access device with an arm in position;
Fig. 49(j) is a view of a device similar to Fig. 49 (i) with a lip seal; and
Fig. 49 (k) is a view of a device similar to Fig. 49(i) with another lip seal.
Fig. 50 is a perspective view of a hand access device according to the invention in use;
Fig. 51 is a perspective view of the device of Fig. 50 in use with a hand being pushed through the device;
Fig. 52 to 54 are side cross sectional views of the device of Figs. 50 and 51 with a surgeon's hand being progressively inserted through the device;
Figs. 54 (a) to 54 (d) are views illustrating an assembly of a hand access device;
Figs. 55 (a) to (c) are, respectively, plan, side and side cross sectional views of the device of Figs. 50 to 54 in a closed configuration;
Figs. 56 (a) to (c) are views similar to Fig. 55 with the device partially open;
Figs. 57 (a) to (c) are views similar to Fig. 55 with the device closed;
Fig. 58 (a) to 60 (c) are views similar to Figs. 55 (a) to 57 (c) of another device according to the invention;
Fig. 61 is a cross sectional view of the device of Figs. 50 to 57 (c) mounted on a retractor;
Fig. 62 is a cross sectional view of the device of Figs. 50 to 57 (c) being mounted on another retractor.
Fig. 63 is a cross sectional view of the device, fully assembled to the retractor of Fig. 62.
Fig. 64 is a perspective view of another hand access device;
Fig. 65 is a perspective view of the device of Fig. 64 with a hand being inserted;
Figs. 66 and 67 are perspective views of hand access devices;
Fig. 68 is a cross sectional view of the hand access device of Figs. 64 and 65 mounted on a retractor with excess retractor sleeve and a lip seal;
Fig. 69 is a cross sectional view of the device of Fig. 68 with an arm in place;
Fig. 70 is a view of another arrangement similar to that of Figs. 68 and 69;
Fig. 71 is an exploded perspective view of an assembly of the invention comprising a retractor and an iris valve;
Fig. 72 is a cross sectional view of the device of Fig. 71 assembled and in position in an incision;
Fig. 73 is a top plan view of the device of Fig. 72 with the iris closed;
Fig. 74 is a reverse plan view of the device of Fig. 72 in the configuration of Fig. 73;
Fig. 75 is a top plan view of the device of Fig. 72 with the iris open; and
Fig. 76 is a reverse plan view of the device of Fig. 72 in the configuration of Fig. 75.

### Detailed Description

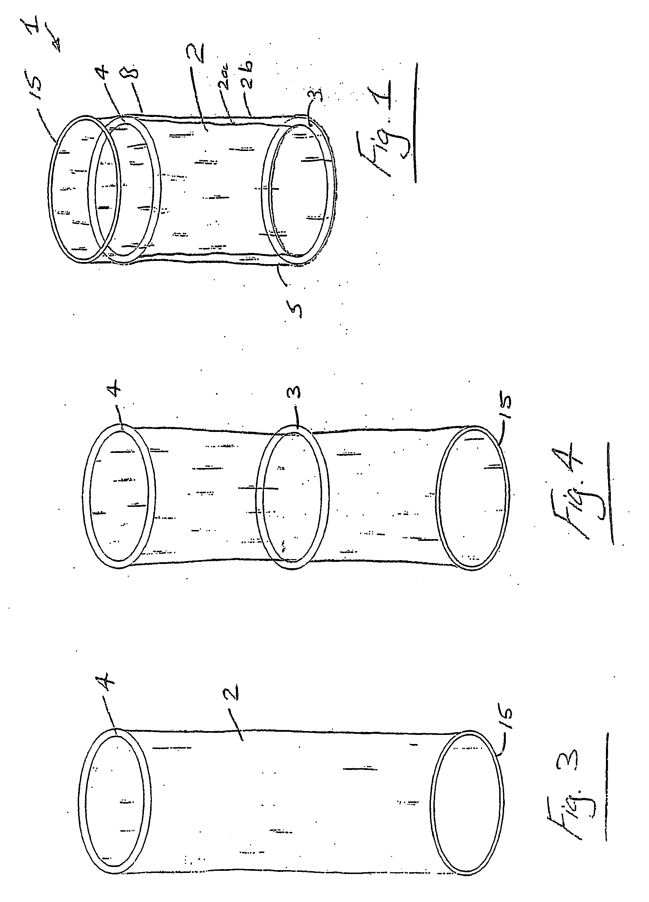

Figs 1 to 10, 11 to 19, 20 to 22, 23 to 28, 29 to 33, 34 to 35, 42 to 45, 46 to 49, 49 (a), 62 to 63, 64 to 67 and 68 to 70 below are presented as examples which may be useful for the understanding of the invention.

Referring to the drawings, and initially to Figs. 1 to 10 thereof there is illustrated a medical device 1 comprising a retractor member provided by a sleeve 2, a distal member provided by a distal ring 3 of resilient material such as an O-ring and a proximal member provided by a proximal ring 4 which may also be an O-ring.

The sleeve 2 is of any suitable material such as of pliable plastics film material and comprises a distal portion 5 for insertion through an incision 6, in this case made in a patient's abdomen 7, and a proximal portion 8 for extending from the incision 6 and outside of the patient.

In this case the distal ring 3 is not fixed to the sleeve 2 but rather the sleeve is led around the ring 3 and is free to move axially relative to the distal ring 3 somewhat in the manner of a pulley. The proximal ring 4 is fixed to the sleeve 2, in this case at the proximal inner end thereof. The sleeve 2 terminates in a handle or gripping portion which in this case is reinforced by a gripping ring 15.

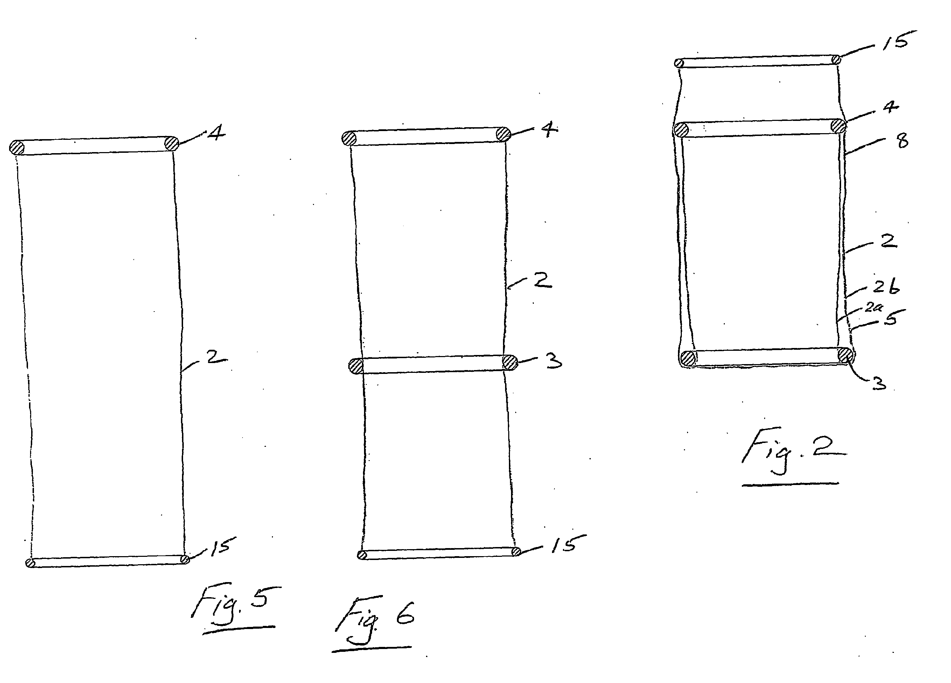

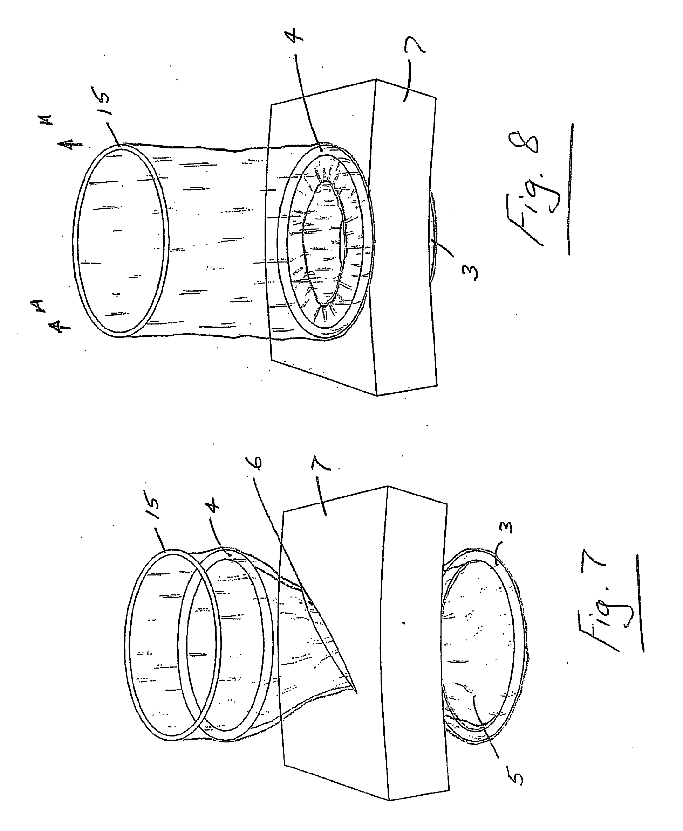

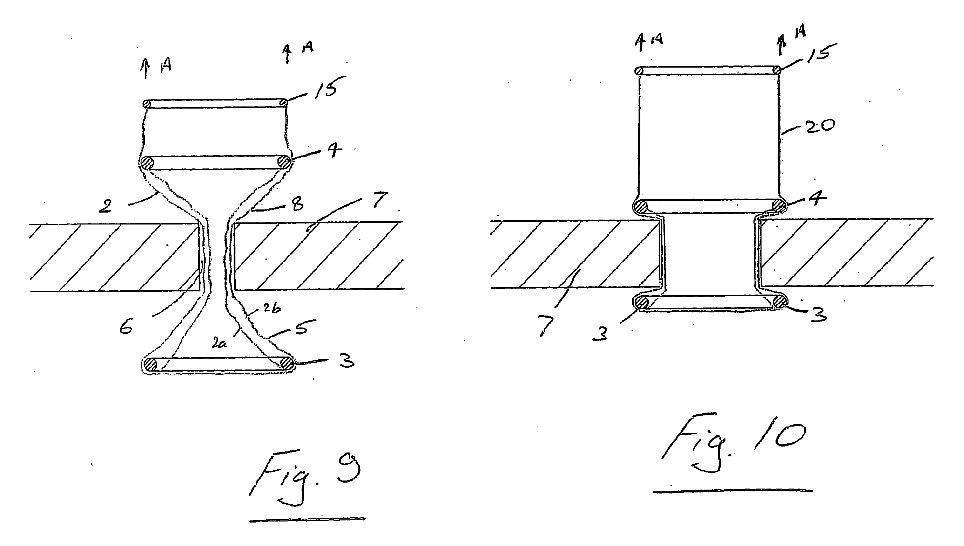

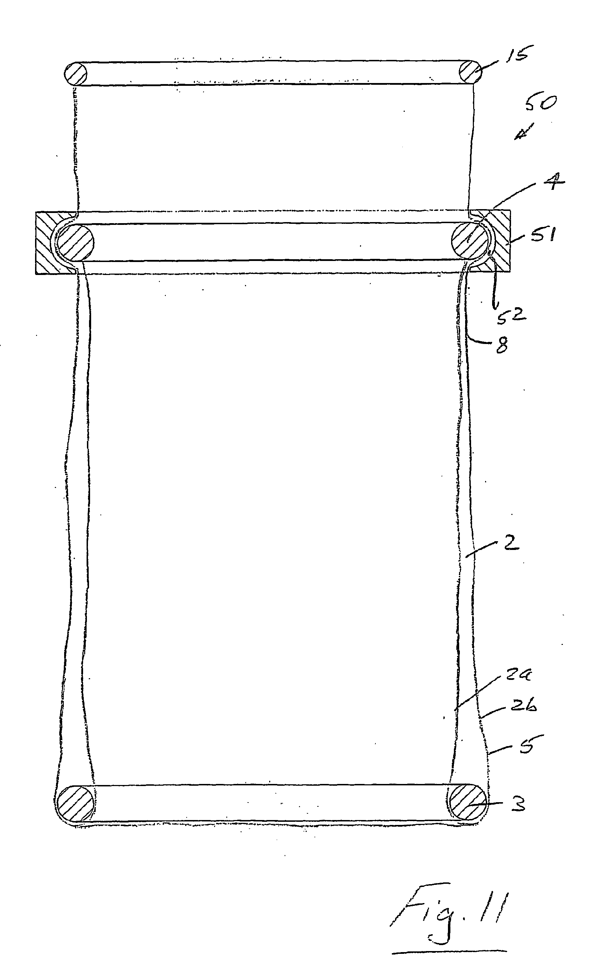

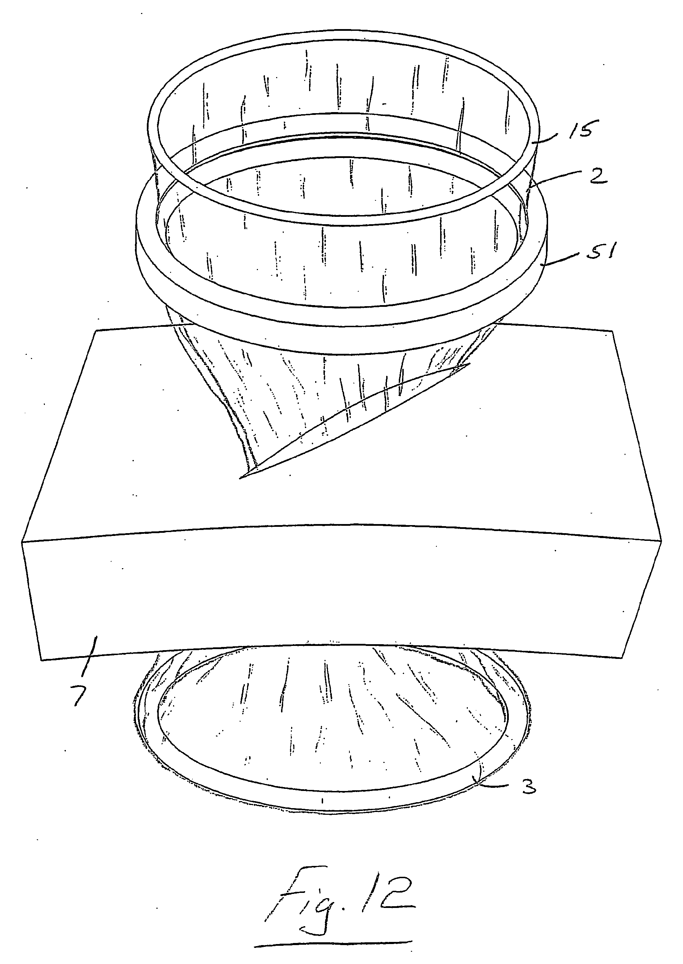

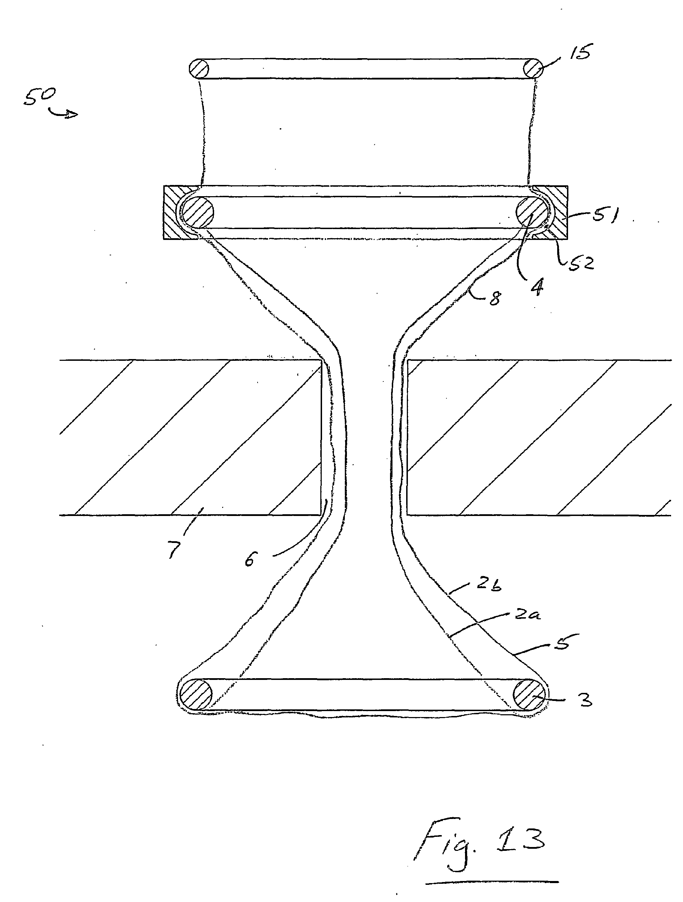

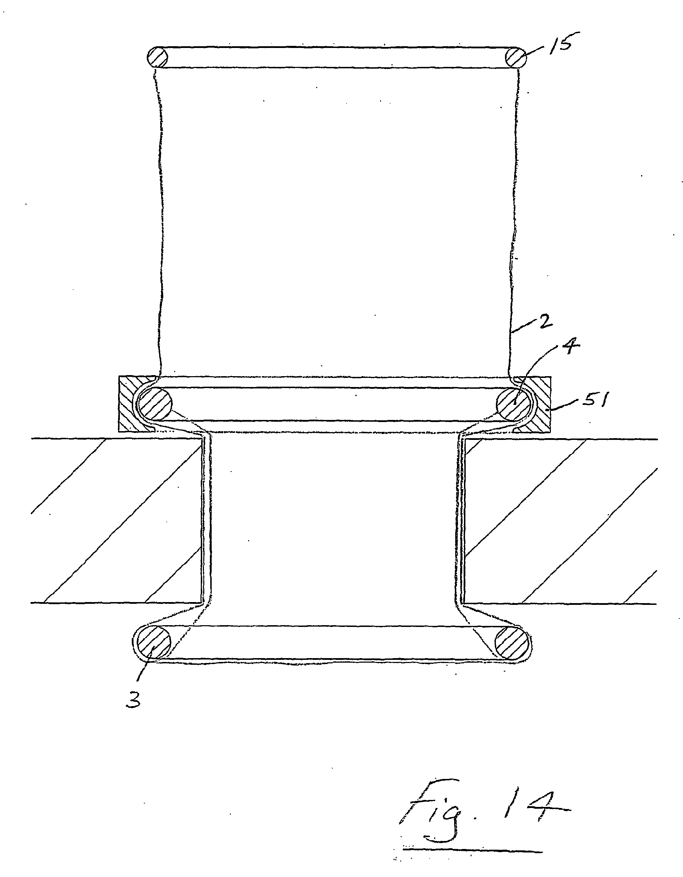

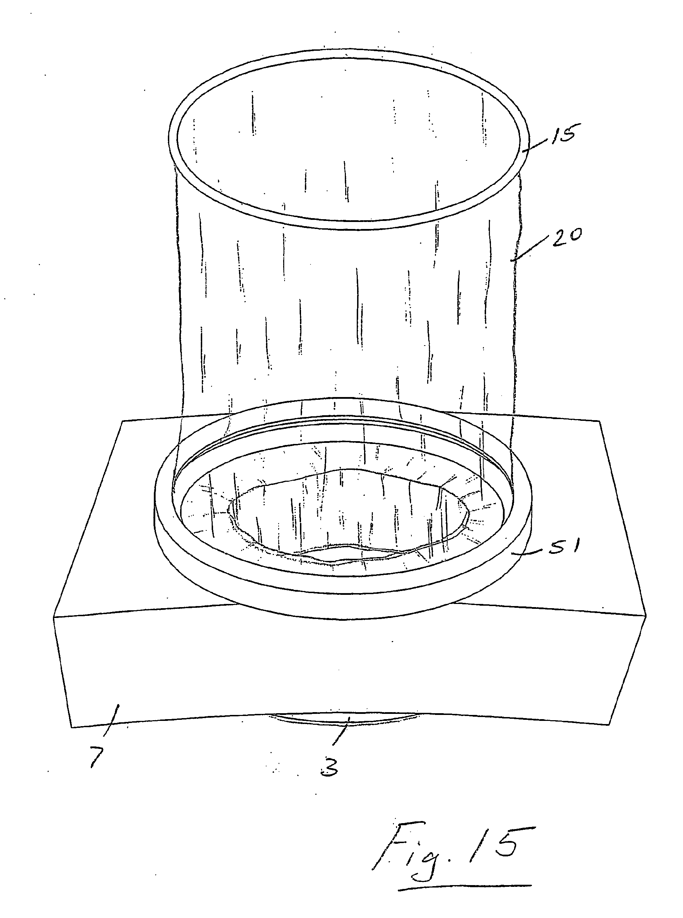

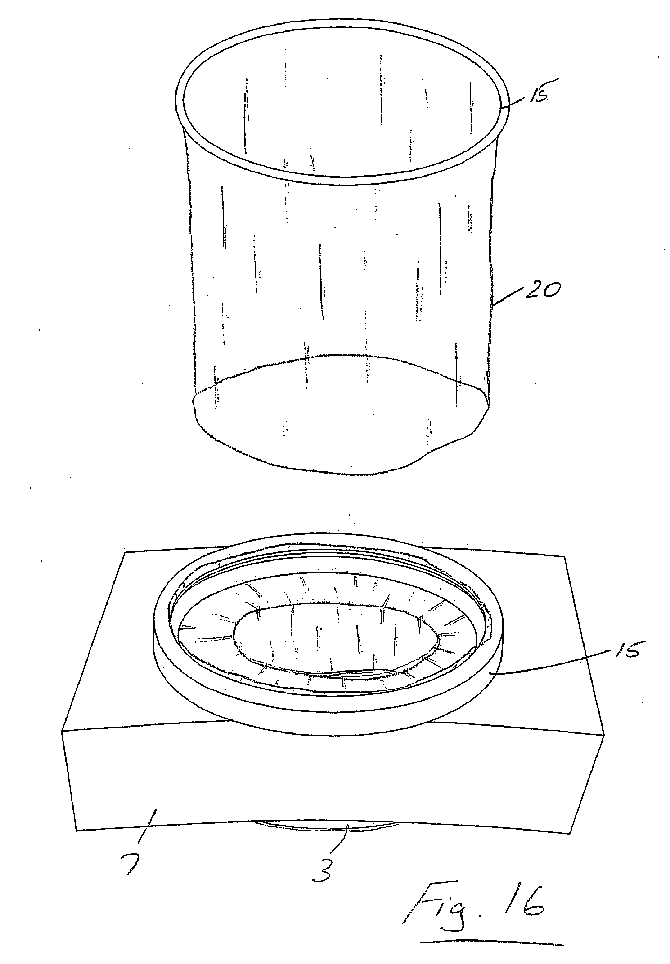

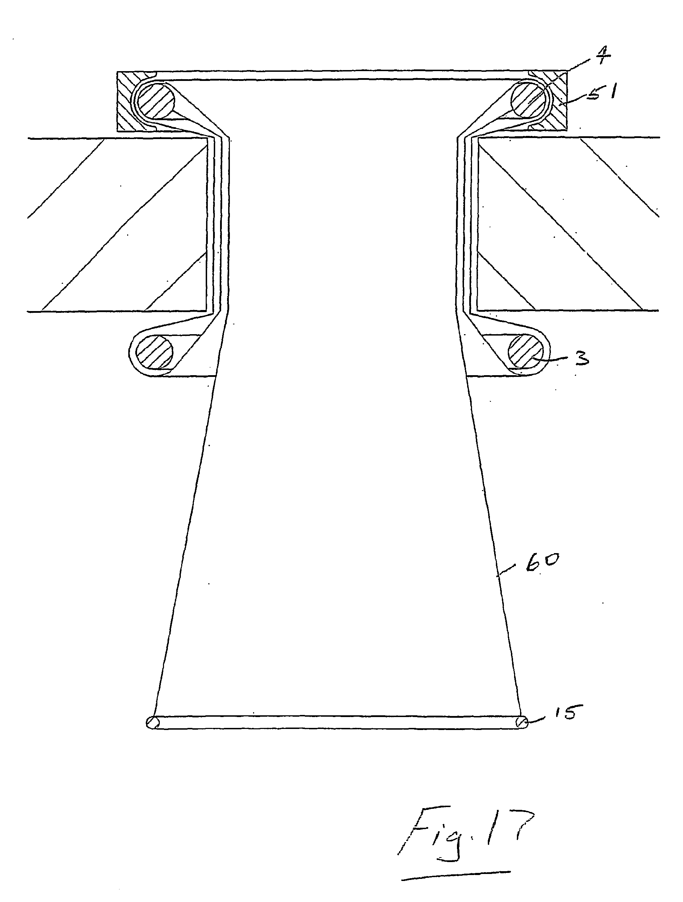

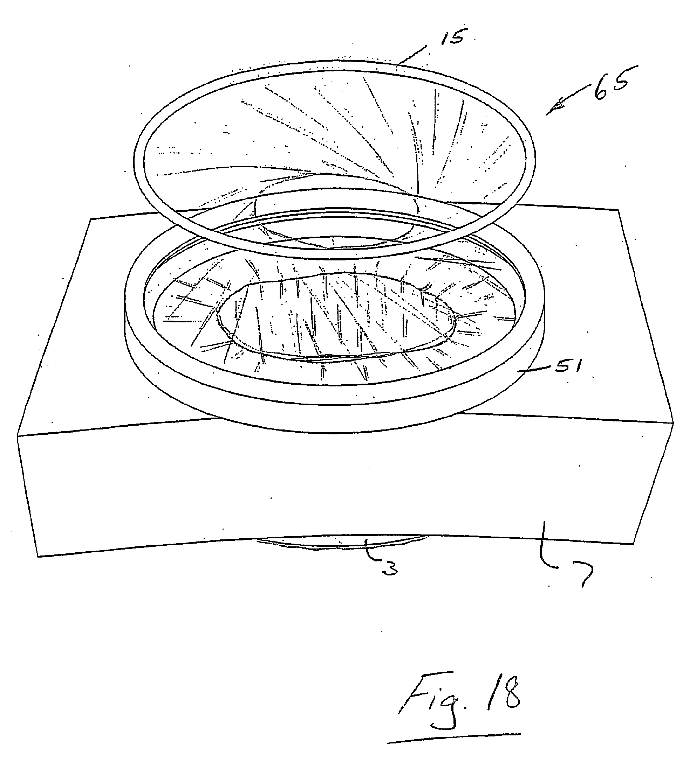

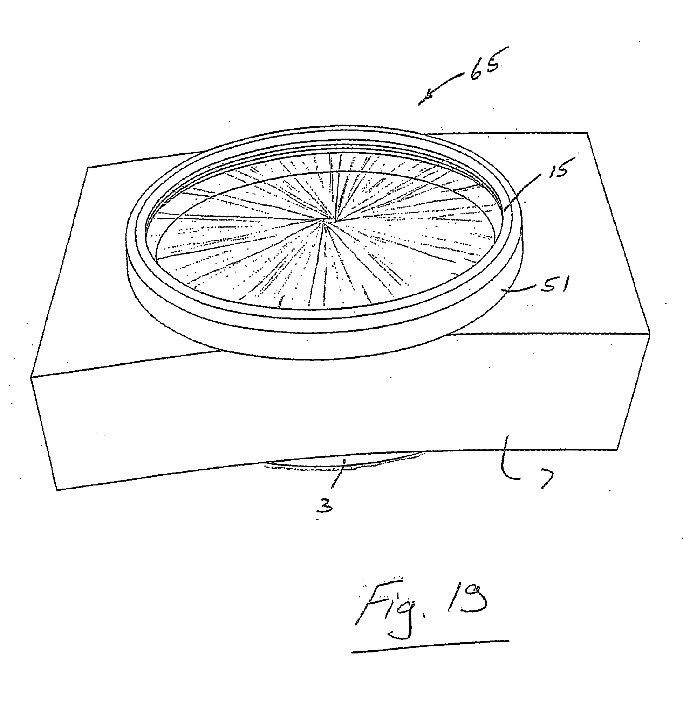

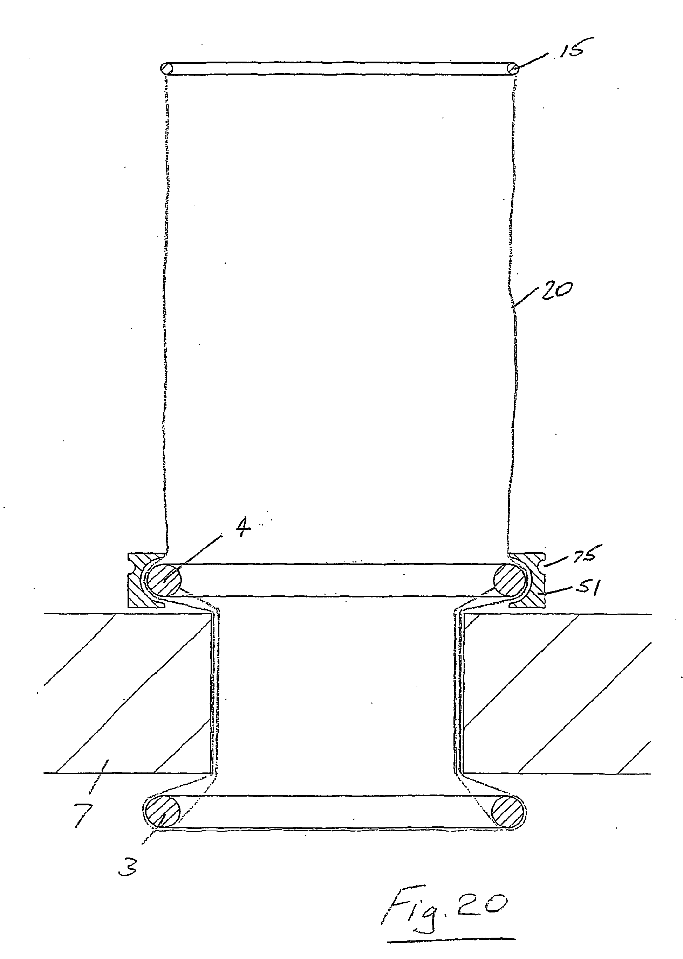

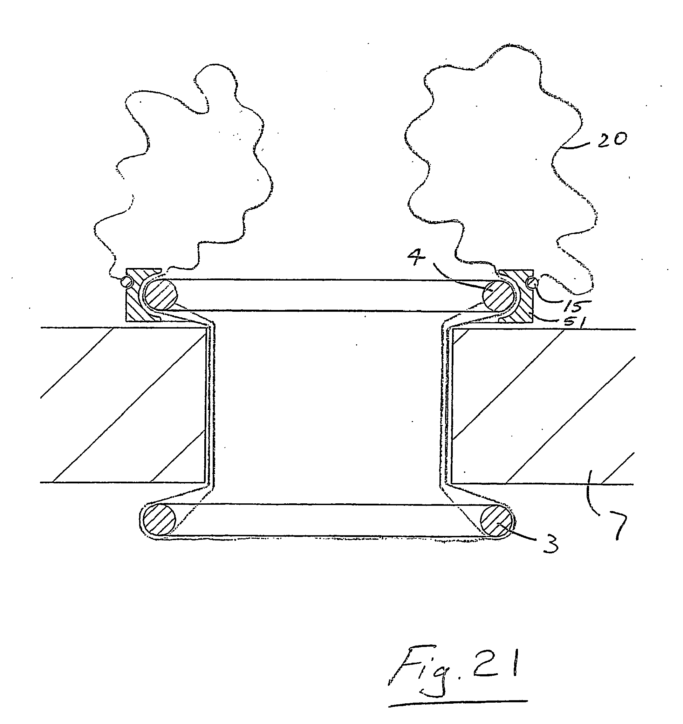

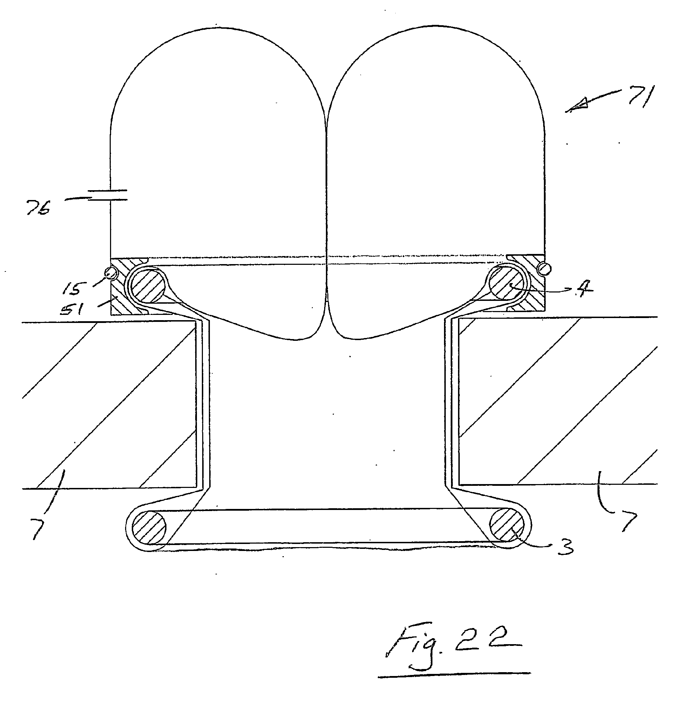

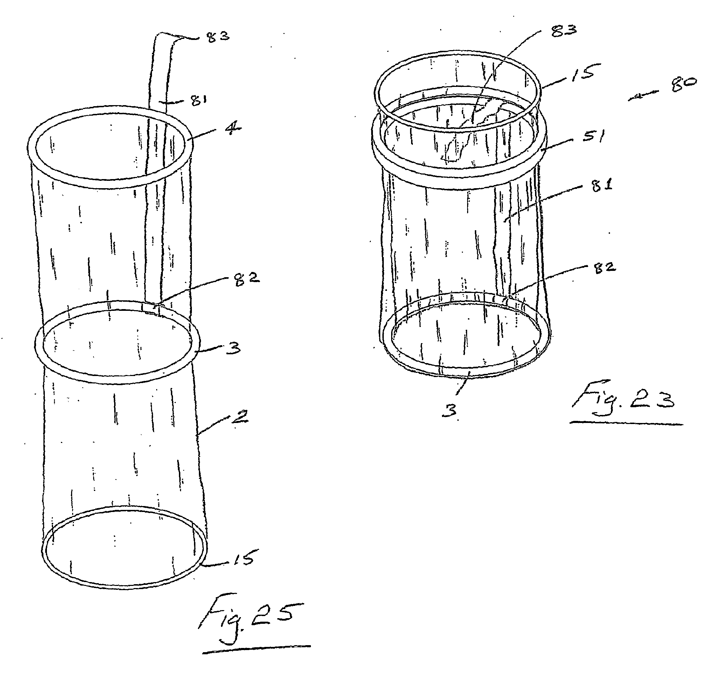

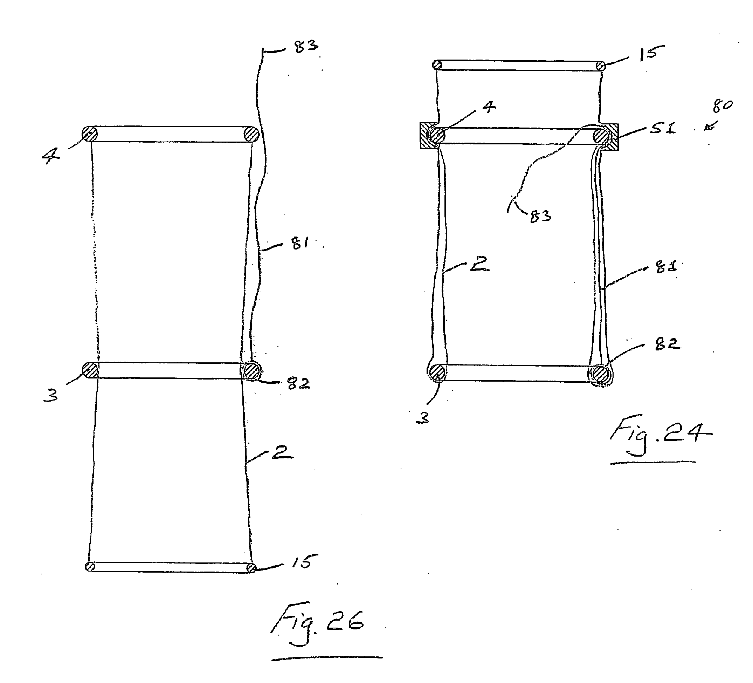

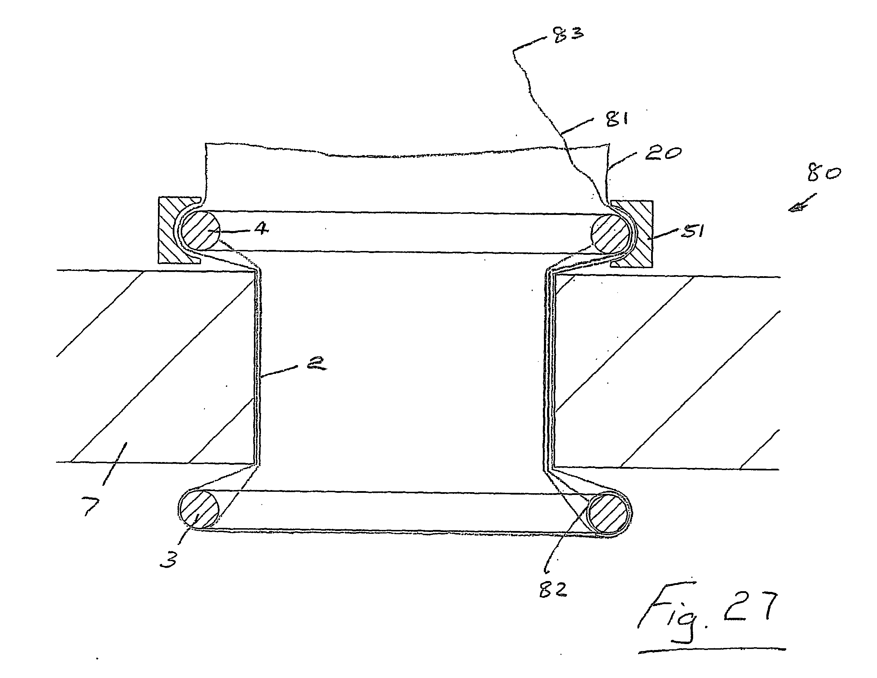

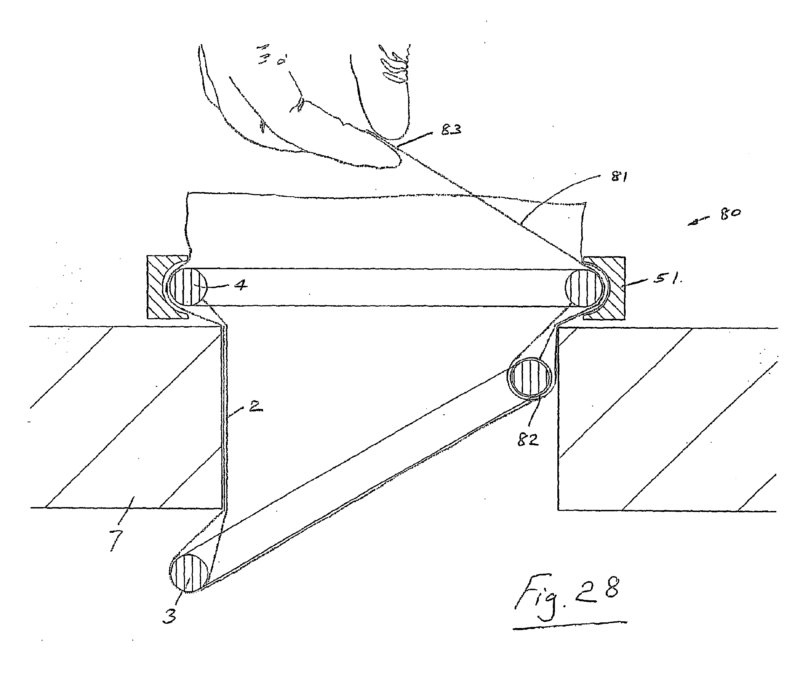

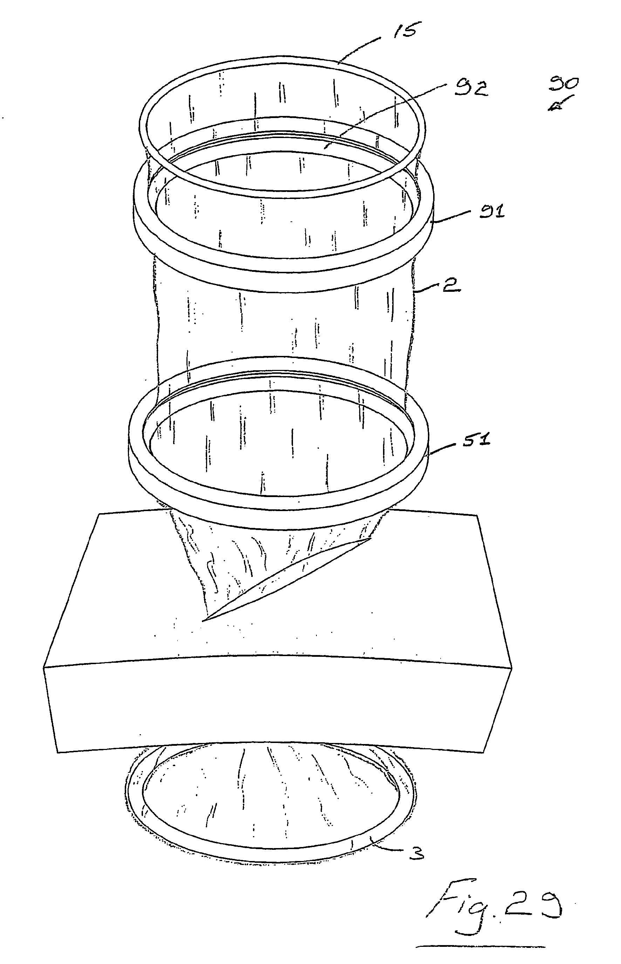

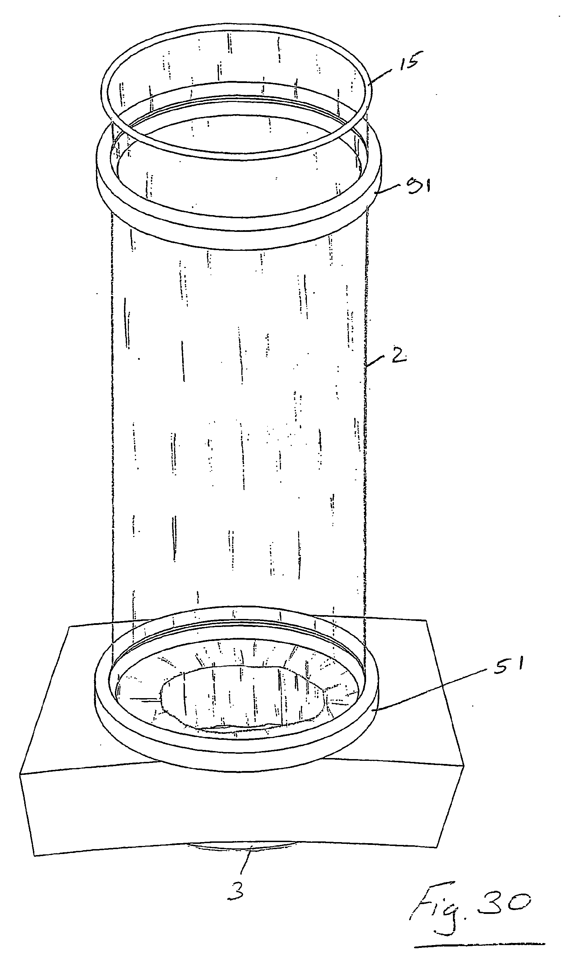

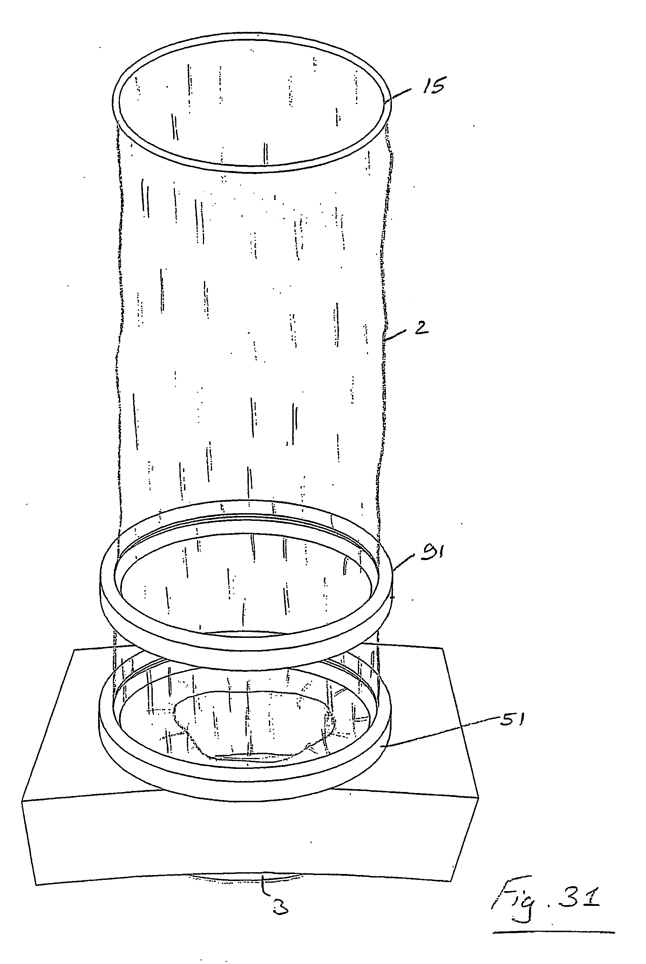

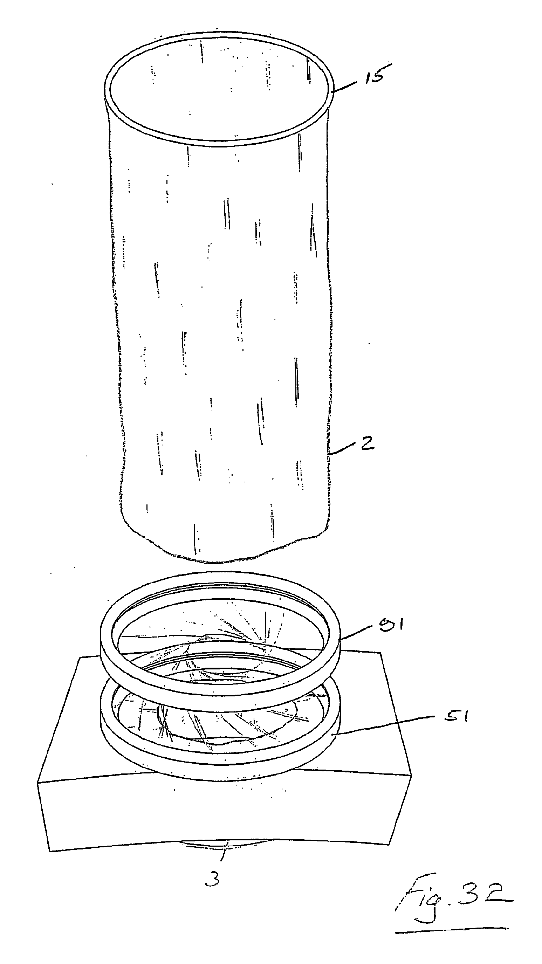

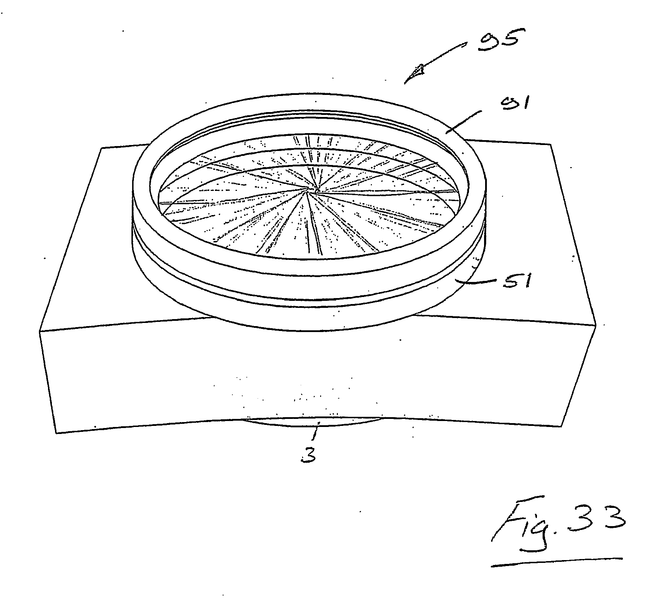

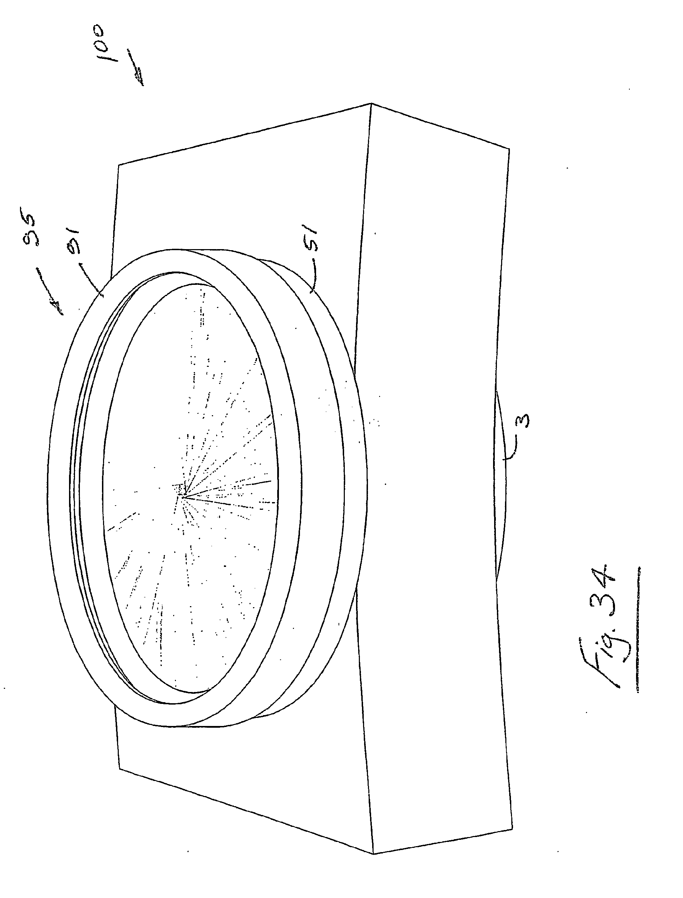

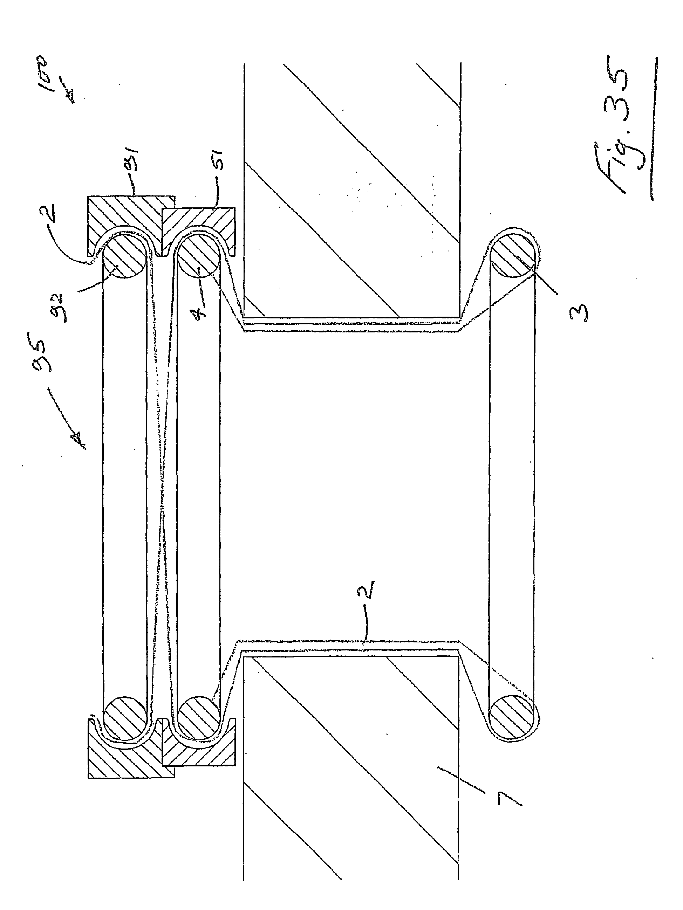

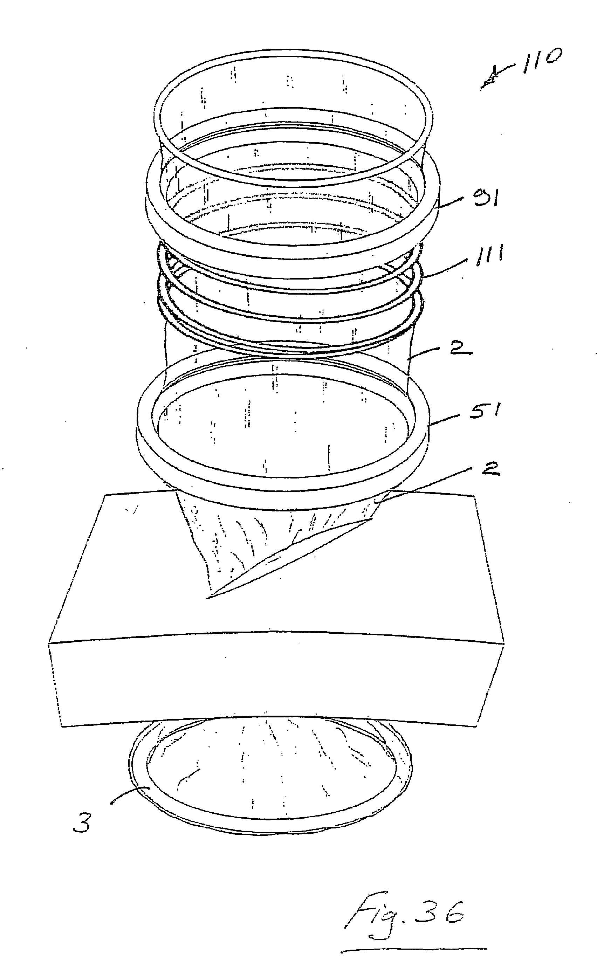

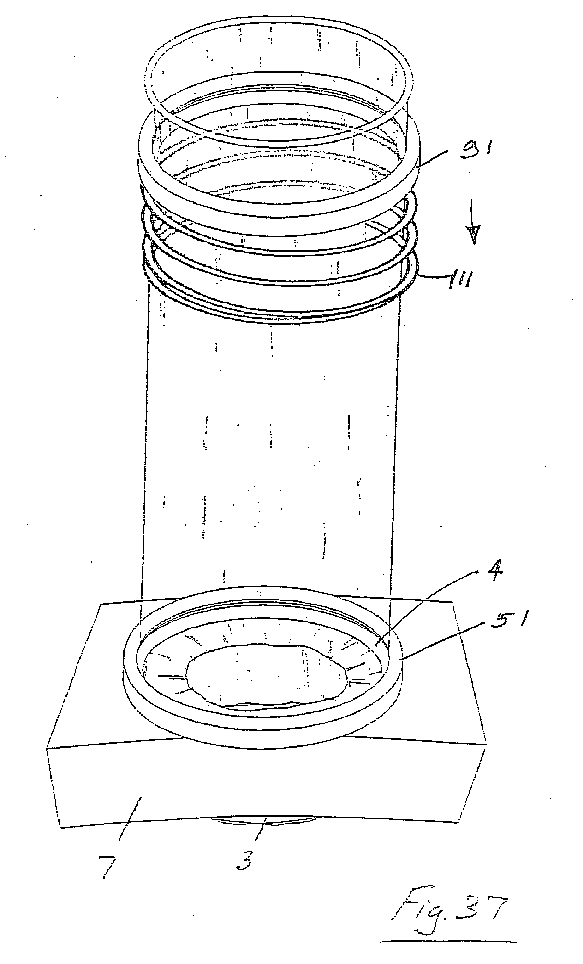

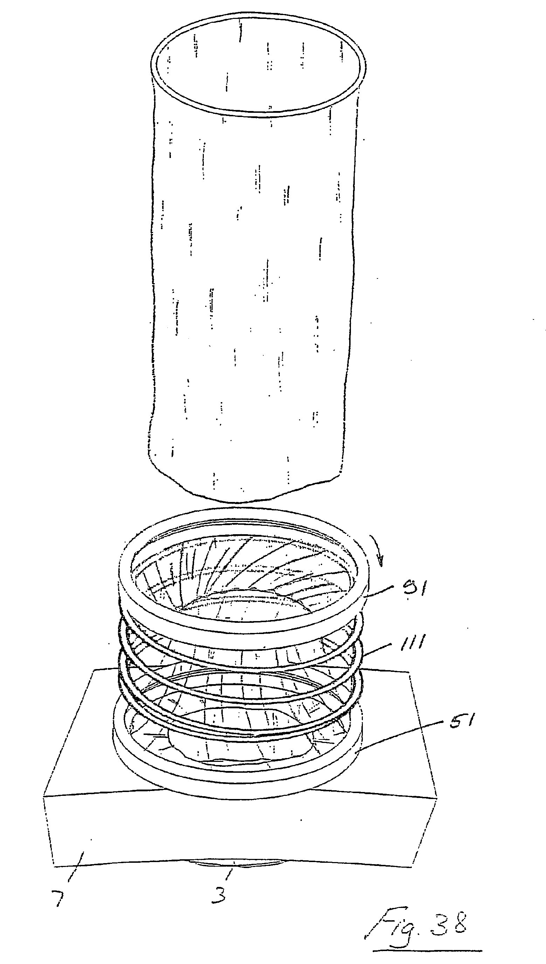

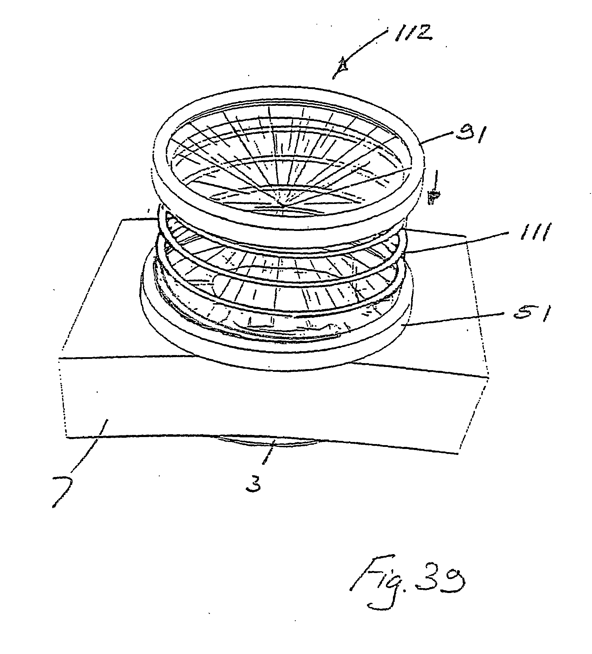

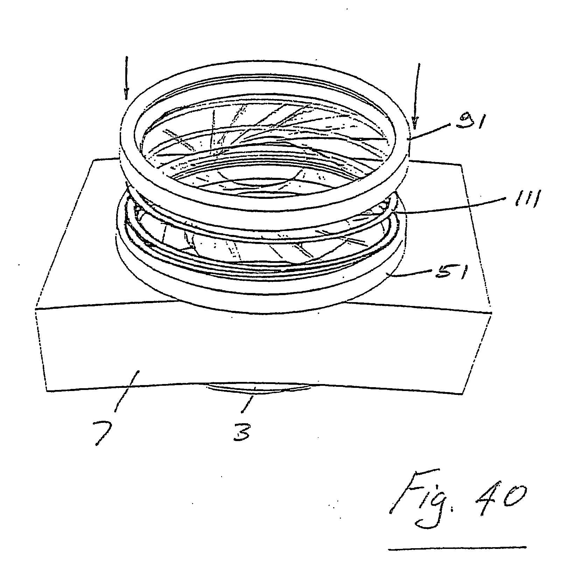

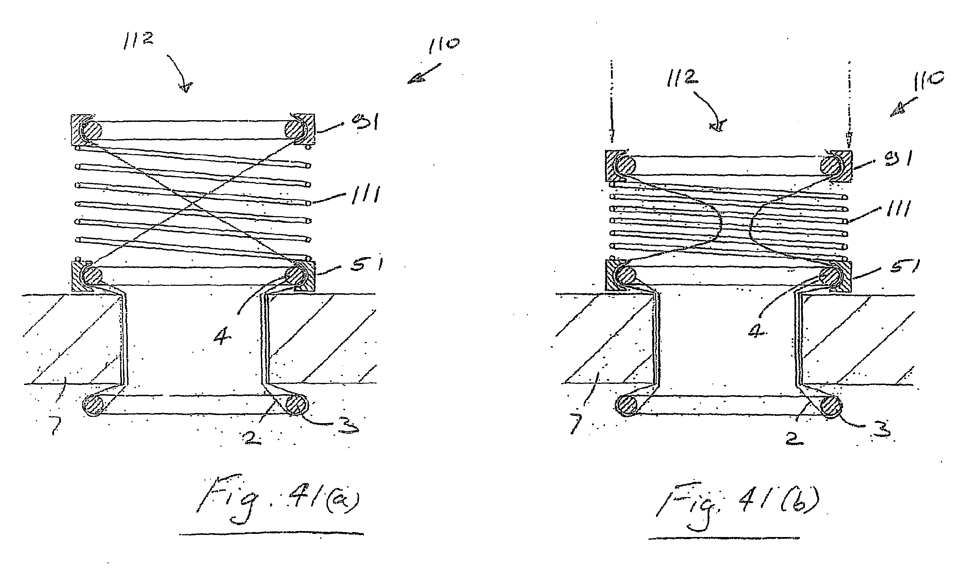

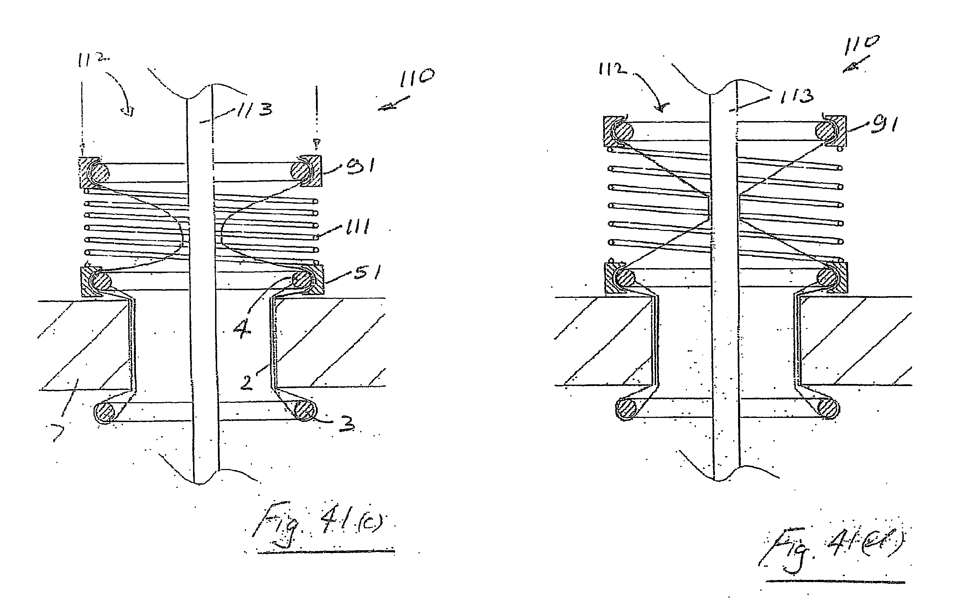

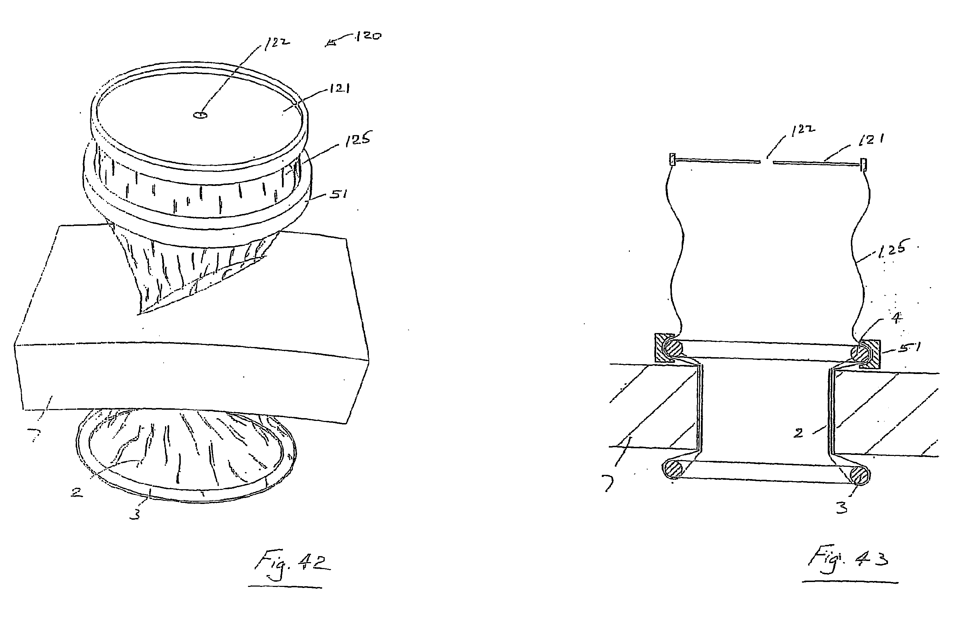

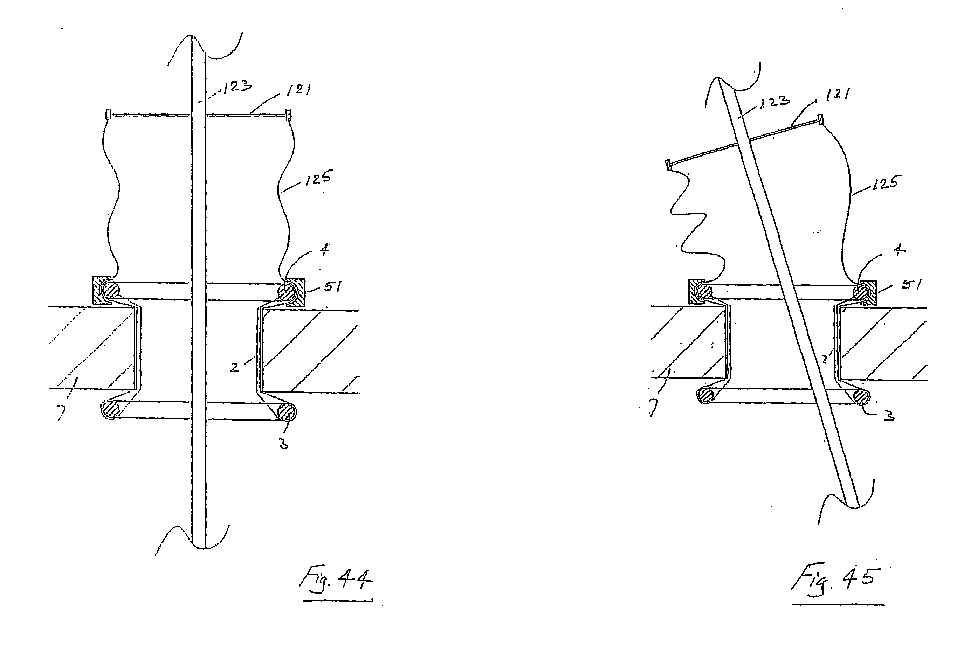

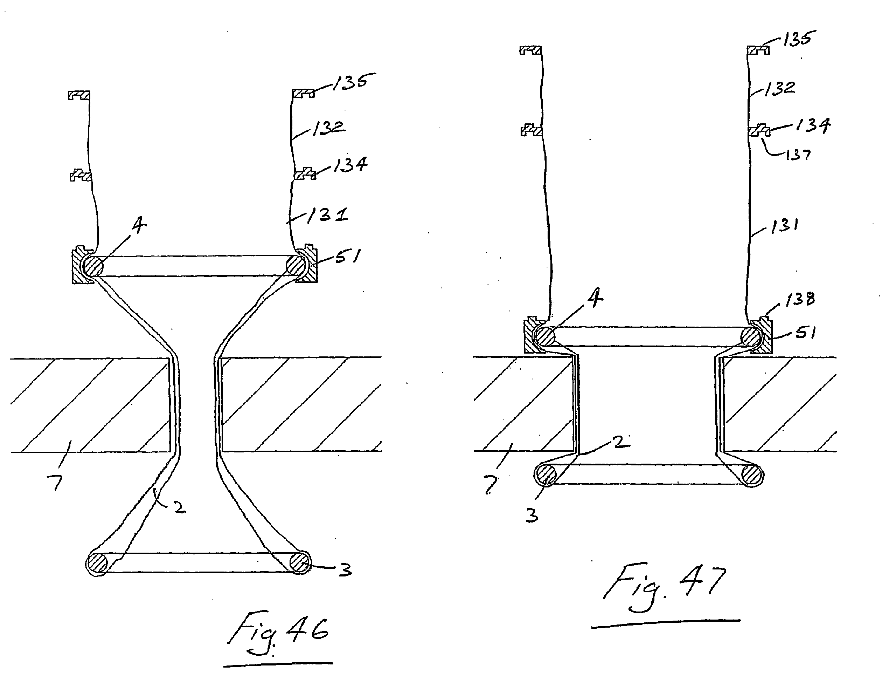

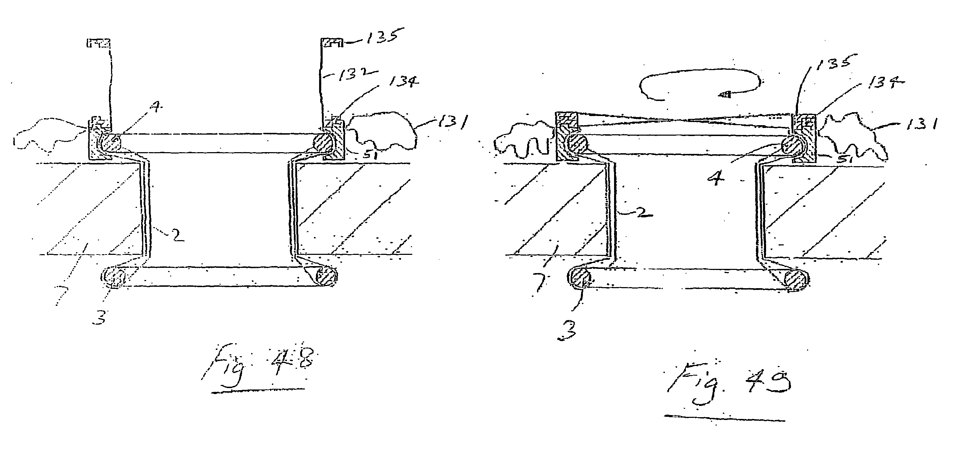

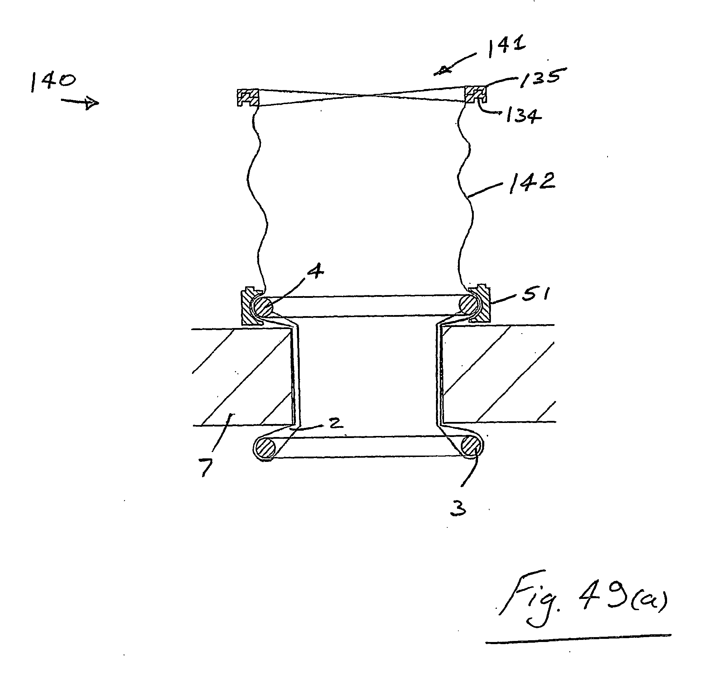

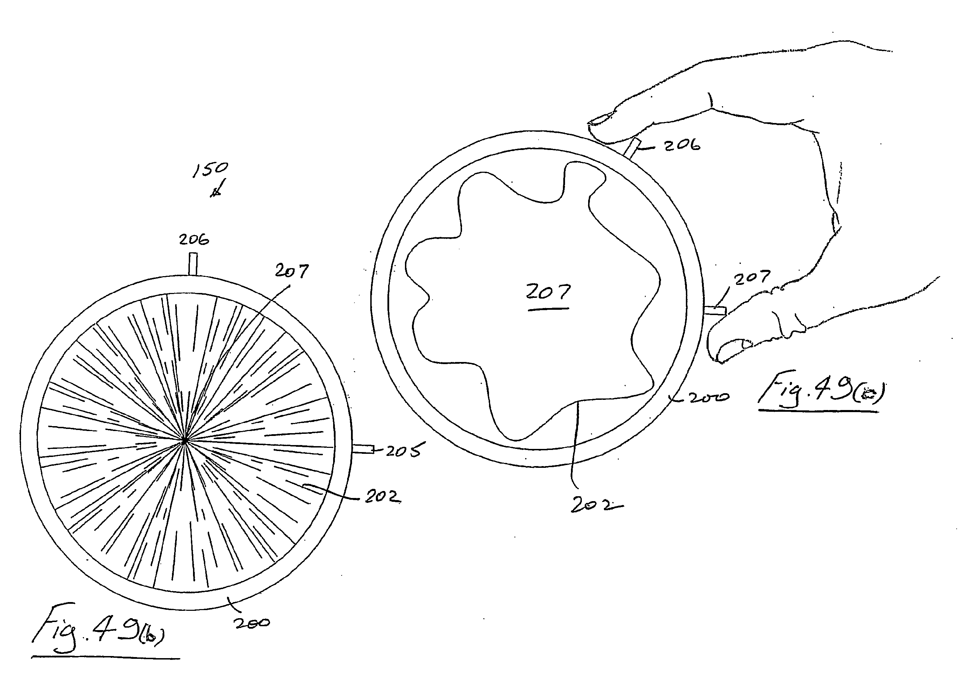

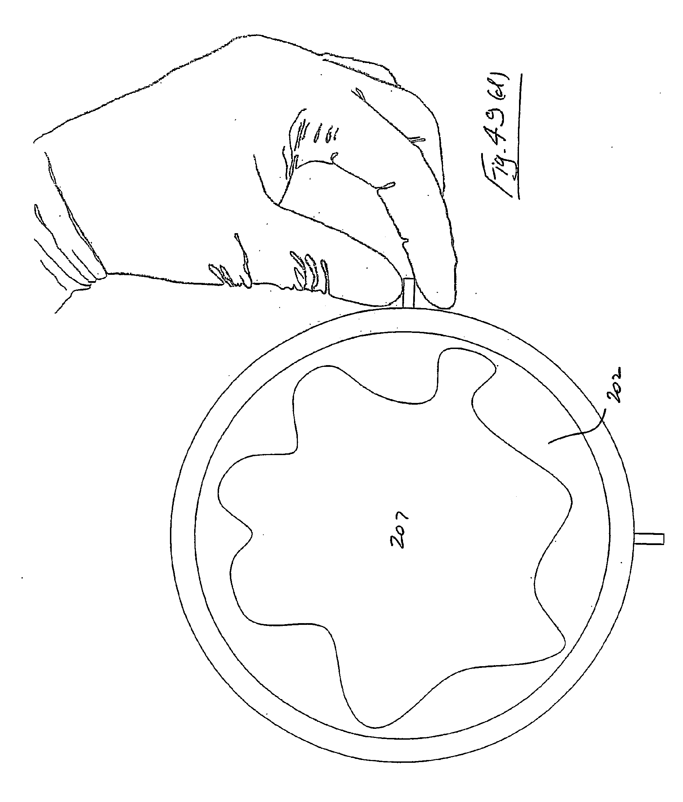

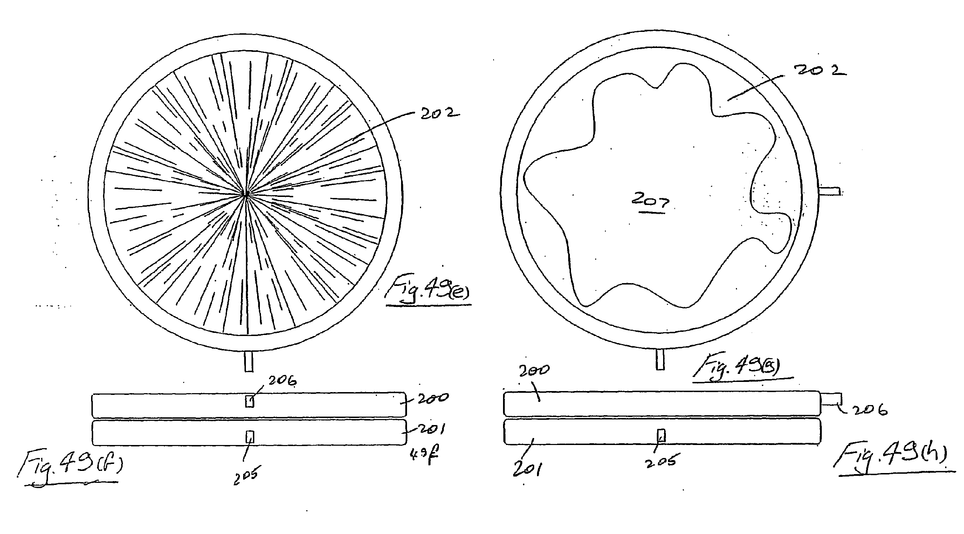

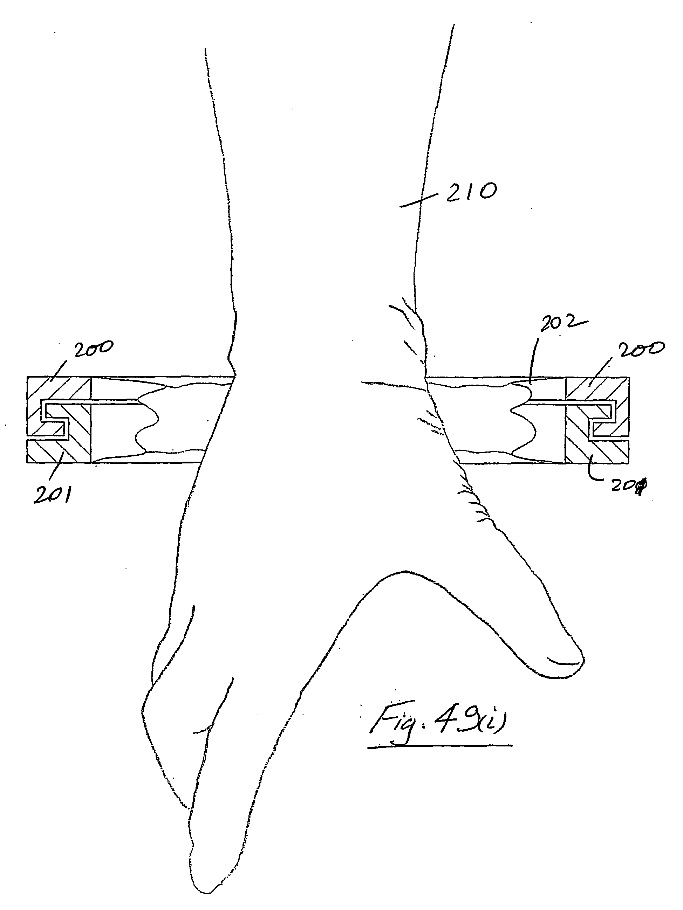

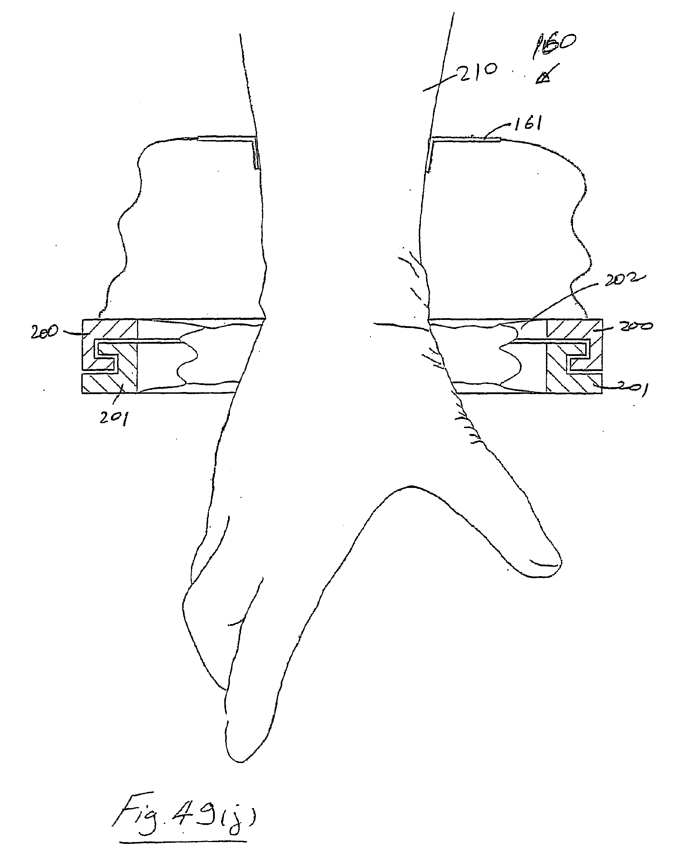

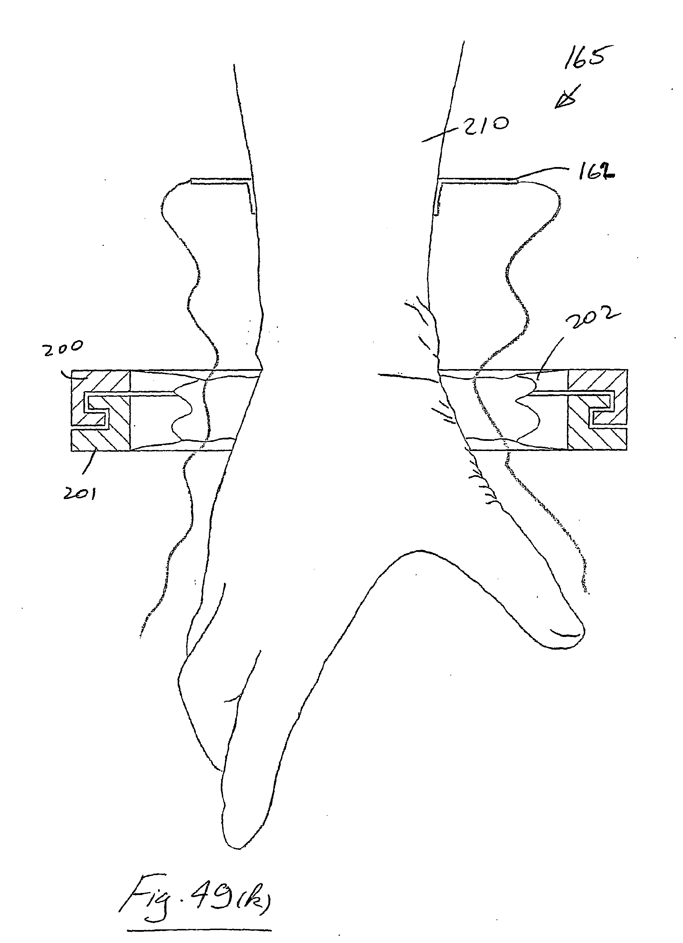

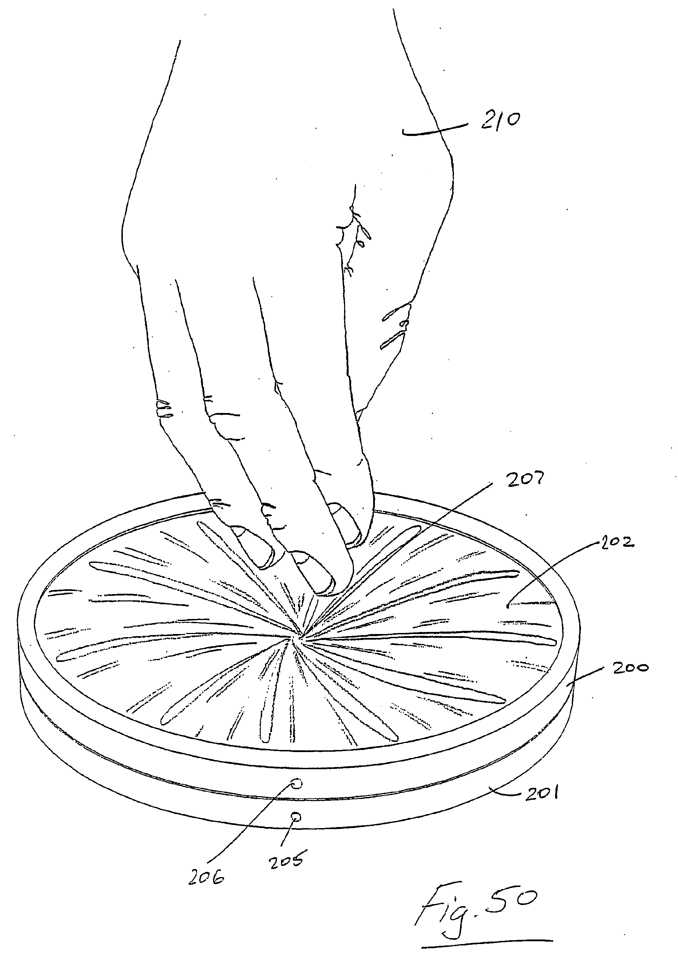

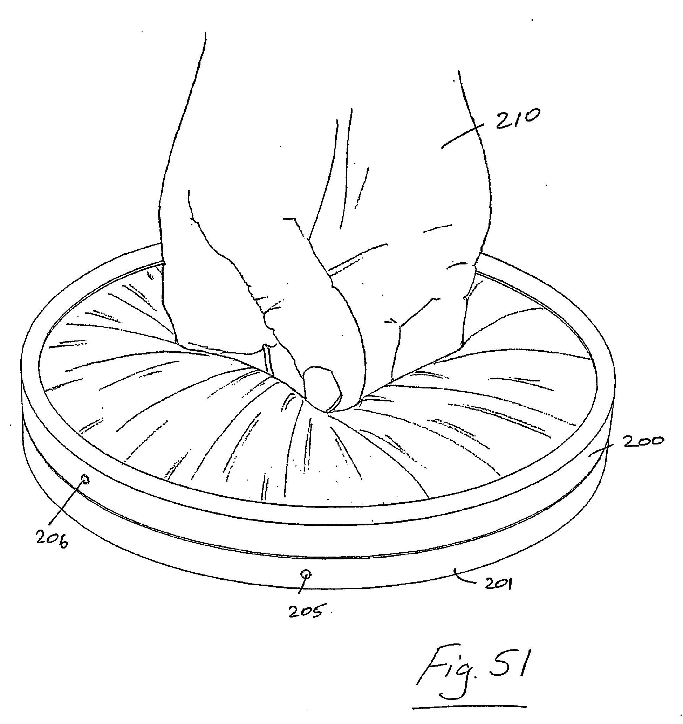

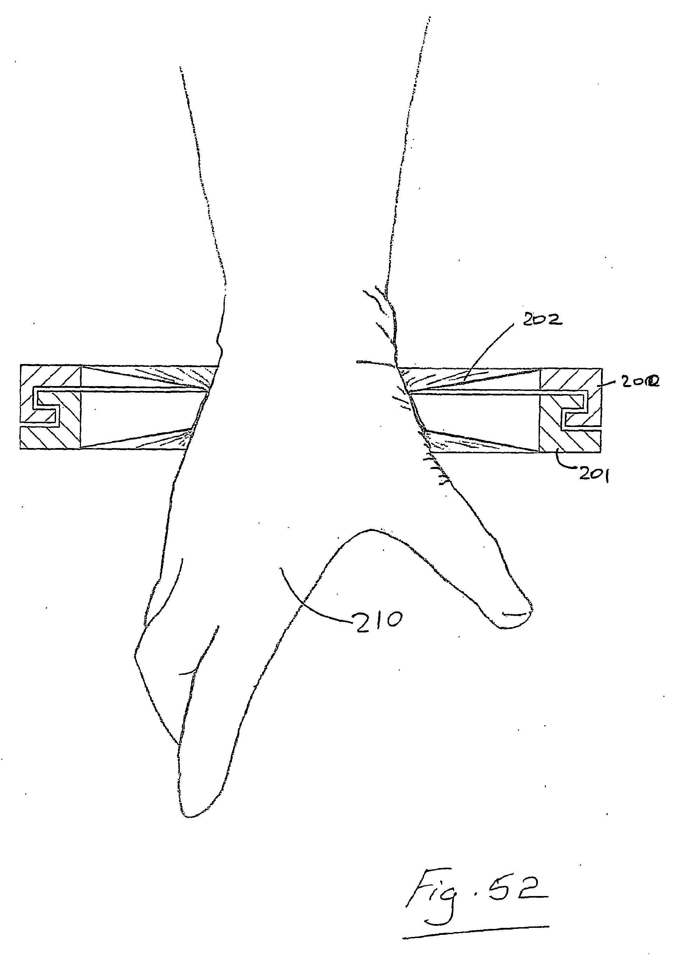

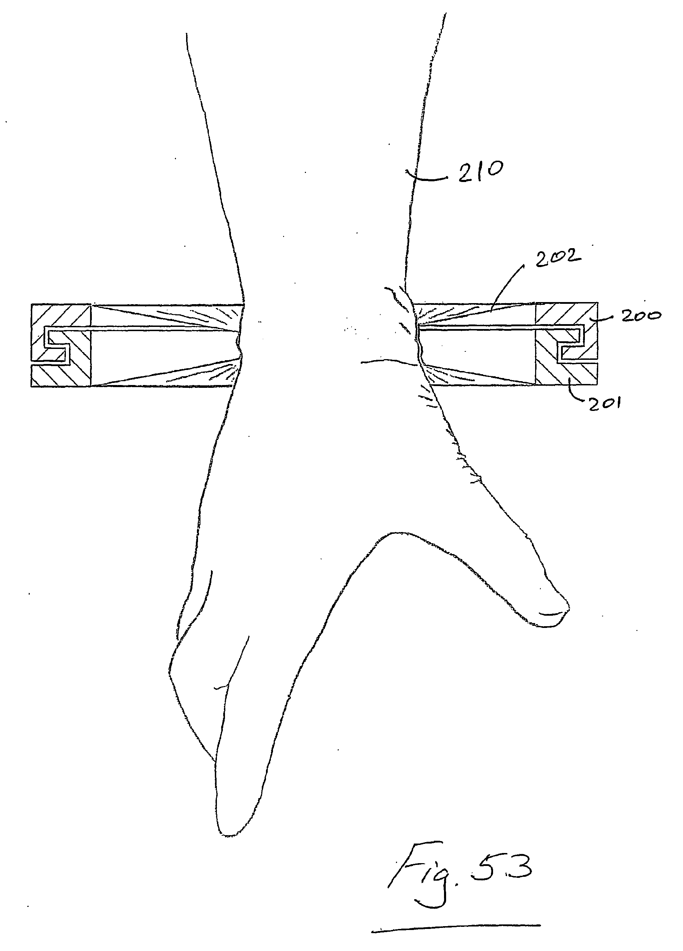

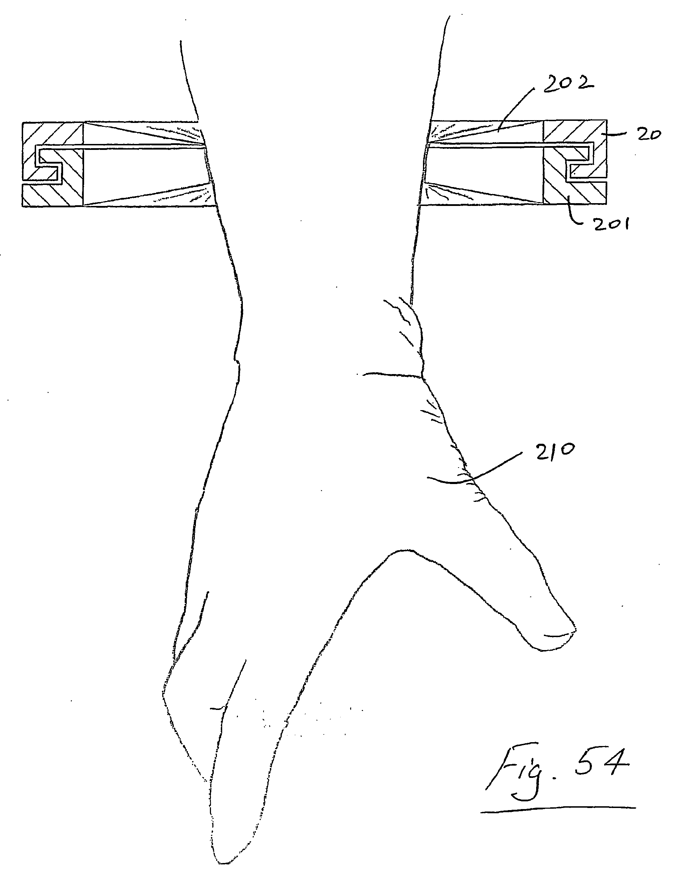

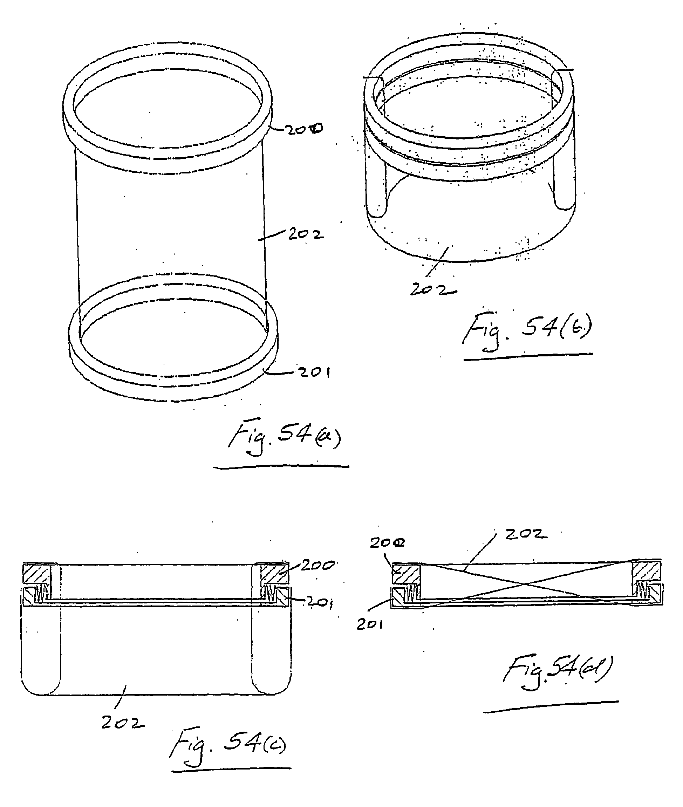

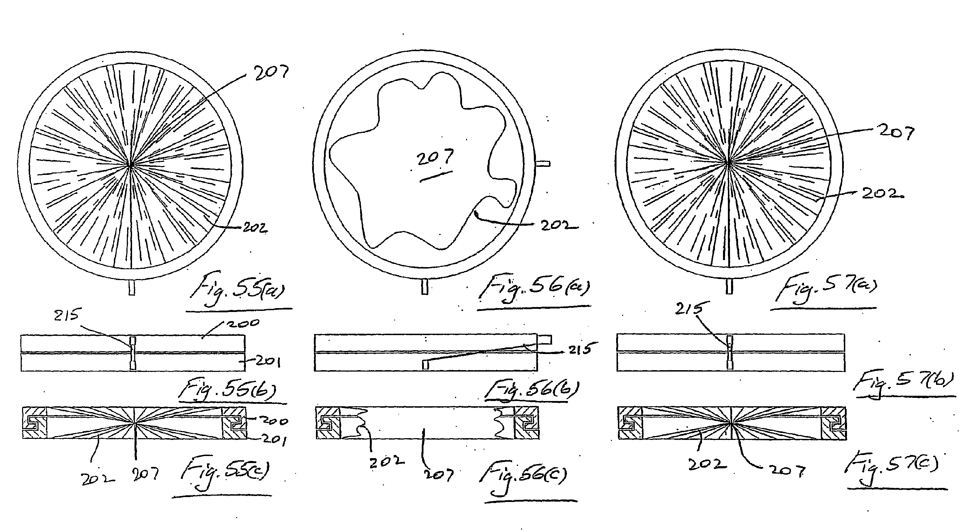

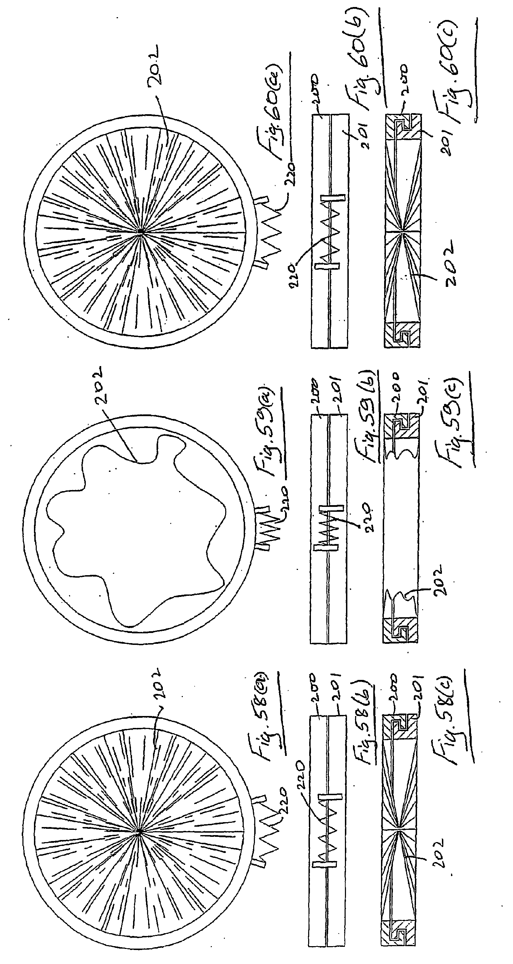

To configure the retractor device according to the invention a sleeve 2 is first provided with the gripping ring 15 fixed at one end and the proximal ring 4 fixed at the other end [Figs. 3, 5]. The distal ring 3 is then placed over the sleeve 2 as illustrated in Figs. 4 and 6. The gripping ring 15 is then used to manipulate the sleeve 2 so that the sleeve 2 is folded back on itself into the configuration of Figs. 1 and 2 in which the gripping ring 15 is uppermost. The sleeve extends from the proximal ring 4 and the distal ring 3 is contained between inner and outer layers 2a, 2b of the sleeve 2. The device is now ready for use.

The resilient distal ring 3 is scrunched up and inserted through the incision 6 with the distal end 5 of the sleeve 2 as illustrated in Fig. 4. The sleeve 2 is then pulled upwardly in the direction of the-arrows A in Figs. 8 to 10. On pulling of the sleeve 2 upwardly the outer layer 2b is pulled up while the inner layer 2a is drawn around the proximal ring 3. This results in shortening the axial extent between the proximal ring 4 and the distal ring 3, tensioning the sleeve and applying a retraction force to the margins of the incision 6. The system appears to be self locking because we have observed that when tension is applied to the sleeve 2 and the pulling force is released the rings 3, 4 remain in position with a retraction force applied. Frictional engagement between the layers of the sleeve in this configuration may contribute to this self locking.

As the incision is being retracted the margins are also protected by the sleeve. On retraction, an access port is provided, for example for a surgeon to insert his hand and/or an instrument to perform a procedure.

Excess sleeve portion 20 outside the incision may, for example, be cut-away.

The retractor is suitable for a range of incision sizes and is easily manufactured. It is also relatively easy to manipulate, in use.

Referring now to Figs. 11 to 19 there is illustrated another device 50 which is similar to the device described above with reference to Figs. 1 to 10 and like parts are assigned the same reference numerals. In this case the device comprises a guide member 51 for the proximal ring 4. The guide member 51 is in the form of an annular ring member with an inwardly facing C-shaped groove 52 which is sized to accommodate the ring 4 as illustrated. The outer layer of the sleeve 2 is interposed between the ring 4 and the guide 51 to further control the pulling of the sleeve and thereby further controlling the application of the retraction force. The guide 51 also assists in stabilising the proximal ring 4. The use of the device 50 is illustrated in Figs. 12 to 15 is similar to that described above.
Referring to Fig.16, it will be noted that in one case the excess sleeve portion 20 may be cut-away.

Referring to Fig. 17, in this case the excess sleeve portion is inverted 60 into the incision. In this configuration it may act as an organ retractor, or provide the surgeon with an open tunnel to work in.

Referring to Figs. 18 and 19 in this case the excess sleeve portion is twisted to form an iris diaphragm valve 65.

In the embodiment illustrated in Figs. 20 to 22 a device 70 has an integral seal/valve 71. The device 70 is similar to that described above with reference to Figs. 11 to 19 and like parts are assigned the same reference numerals. In this case the guide member 50 has an outer groove 75 to receive the gripping ring 15 as illustrated in Figs. 21. The excess sleeve portion 20 is folded out and down and the gripping ring 15 is engaged in the groove 75 to provide an air tight seal. In this configuration the excess sleeve may be inflated through an inflation port 76 [Fig. 22] to provide an integral access valve 71. The valve may be used to sealingly engage a hand, instrument or the like passing therethrough. The inflated sleeve portion defining the valve is evertable on passing an object therethrough.

Referring to Figs. 23 to 28 there is illustrated another retractor 80 which is similar to the retractors described above and like parts are assigned the same reference numerals. In this case the retractor 80 has a release mechanism which in this case is provided by a release cord or ribbon 81 which is coupled at one end 82 to the inner ring 3 and terminates in an outer free end 83 which may be grasped by a user. The ribbon 81, on assembly, is led through the gap between the proximal ring 4 and the outer guide member 51 so that it is positioned between the ring 4 and the guide member. The ribbon 81 facilitates release of the self locked sleeve in the in-use configuration sited in an incision. Pulling on the ribbon 81 pulls on the inner ring 3, allowing the ring 3 to be released from the inner wall of the incision to thereby release the device. The flexibility of the ring 3 facilitates this movement.

The advantage of this arrangement is that a user can readily release the device from its self locked retracting configuration.

Referring to Figs. 29 to 33 there is illustrated another device 90 in which parts similar to those of the devices described above are assigned the same reference numerals. In this case the device 90 has a lower guide ring 51 for the proximal ring 4 and an outer guide assembly provided by an upper guide ring 91 and a second proximal ring 92 between which the sleeve 2 is led. The device is used to first retract an incision as described above. During this phase the outer guide assembly is conveniently external of the guide member 51 and proximal ring 4. Indeed, it may be completely detached from the sleeve 2 and subsequently coupled to the sleeve 2 at an appropriate stage such as when the incision is retracted as illustrated in Fig. 30. The outer guide assembly is then moved downwardly towards the incision as illustrated in Fig. 31. This may be achieved while pulling the sleeve 2 upwardly. When the guide assembly is adjacent to the guide member 51 excess sleeve length may be severed as illustrated in Fig. 32. By twisting the guide assembly relative to the guide member 51 the sleeve 2 is twisted, closing down the lumen of the sleeve 2 and forming an iris type access valve 95 as illustrated in Fig. 33. In this way a sealed access port is provided for hand and/or instrument access through the incision.

It will be appreciated that while reference has been made to an incision made by a surgeon the device may be applied for retraction of any opening such as a body opening.

Referring to Figs. 34 and 35 there is illustrated another retractor device 100 which is similar to the device of Figs. 29 to 33 and like parts are assigned the same reference numerals. In this case a releasable lock is provided to maintain the access valve 95 closed. For interlocking, in this instance the upper guide ring 91 is an interference fit with the lower guide ring 51. Various other locking arrangements may be used such as a screw threaded or bayonet type engagement, magnets, clips and the like.

Referring to Figs. 36 to 41 there is illustrated another retractor device 110 which is similar to the device of Figs. 29 to 33 and like parts are assigned the same reference numerals. In this case the device incorporates a biasing means to bias an integral valve into a closed position. The biasing means is in this case provided by a coil spring 111 which is located around the sleeve between the guide rings 51, 91. In use, the device is used in a similar manner to the device of Figs. 29 to 33 except that on movement of the upper guide ring 91 downwardly the spring 111 also moves downwardly towards the lower guide ring 51, initially into the position illustrated in Fig. 38. Excess sleeve material may be removed at this stage. The spring 111 is tensioned as the upper ring 91 is rotated while pushing the upper ring 91 downwardly. The sleeve material between the two rings 51, 91 is twisted, forming an iris type valve 112 as illustrated in Fig. 39. To open the valve 112 to pass an object such as an instrument, hand, arm or the like therethrough a downward force may be applied to push the upper ring 91 towards the lower ring 51 against the biasing of the spring. This configuration is illustrated in Fig. 40. When the object is inserted the upper ring member 91 is released, allowing the valve to close around the object. The operation of the device 110 will be readily apparent from Figs. 41 (a) to 41 (d). In Fig. 41 (a) the valve 112 is illustrated in a closed resting configuration. Fig. 41 (b) shows the application of a downward force to open the valve 112. An object such as an instrument 113 is shown inserted through the open valve 112 in Fig. 41(c). In Fig. 41 (d) the downward pressure on the upper ring 91 is released allowing the valve 112 to close around the object 113.

Referring now to Figs. 42 to 45 there is illustrated another device 120 which has some aspects similar to the device of Figs. 11 to 18 and like parts are assigned the same reference numerals. In this case the device has a lip seal 121. The lip seal 121 is provided by a membrane with a central aperture 122 through which an object 123 such as an instrument is passed. The lip seal 121 is located on the sleeve 2 proximally of the guide ring 51 such that a proximal flexible sleeve section 125 is provided. This sleeve section 125 is very useful in facilitating offset movements of the object 123 as illustrated in Fig. 45. The sleeve section 125 accommodates movement of the object 123 whilst maintaining sealing engagement between the lip seal 121 and the object 123. It will be appreciated that this feature, as with several other features described above may be utilised in association with other constructions of wound protector/retractors and access parts generally other than those illustrated in the drawings.

Referring to Figs. 46 to 48 there is illustrated another device 130 which has some features similar to those of Figs. 11 to 15, like parts being assigned the same reference numerals. In this case the sleeve has a proximal section external of the wound when the device is in the retracting configuration. This proximal sleeve section comprises a first portion 131 extending from the guide ring 51 and a second portion 132 extending from the first portion 131. The second portion 132 is defined between two spaced-apart iris rings 134, 135. It will be noted that the iris rings 134, 135 have engagement features such as projections and grooves for interengagement on assembly. The iris ring 134 also has an engagement element, in this case provided by a groove 137 for engagement on assembly with a corresponding engagement element of the guide ring 51 which in this case is provided by a projection 138.

The device is fitted as described above to retract an incision, leaving the first and second sleeve portions 131, 132 extending proximally. The first sleeve portion 131 is redundant and can be removed or scrunched up on assembly of the first iris ring 134 to the guide ring 138 as illustrated in Fig. 48. The second or upper iris ring 135 is then rotated to twist the sleeve section 132 to form an iris-type seal as illustrated in Fig. 49. The iris ring 135 is engaged with the iris ring 134 as illustrated to maintain the valve closed.

Referring to Fig. 49(a) there is illustrated another device 140 which has some aspects similar to the device of Figs. 46 and like parts are assigned the same reference numerals. In this case the iris rings 134, 135 are used to form an iris valve 141 which is proximally spaced from the guide ring 51 and a flexible sleeve section 142 is thereby provided between the iris 141 and the guide ring 51. This sleeve section 142 can act as a flexible cannula wall to permit sealed access of a cannula whilst facilitating lateral movement of the cannula somewhat as illustrated in Figs. 44 and 45.

Referring to Figs. 49(b) to 49(i) there is illustrated a surgical device 150 according to the invention comprising a mounting element provided by a first ring element 200 and a second ring element 201. A sealing member which in this case is in the form of a sleeve 202 of pliable material has a first end mounted to the first ring element 200 and a second end mounted to the second ring element 200. For ease of reference the ring elements 200, 201 have associated location markings 205, 206 respectively. The sleeve 202 is twisted and has a normally closed access opening 207 and the sleeve is movable on insertion of a surgeon's hand/arm 210 to open the access opening 207 whilst maintaining substantial sealing engagement with the object.

As will be described in more detail below a biasing means is provided to bias the sleeve into a configuration in which the access opening 207 is normally closed. The biasing may be provided by pre-tensioning the sleeve and/or by using a separate spring element.

Referring to Fig. 49(j) there is illustrated another device 160 which is similar to the device of Figs. 49(d) to (i) and like parts are assigned the same reference numerals. In this case the device has a lip-type seal 161 for further sealing engagement. Another device 165 with a different type of lip seal 162 is illustrated in Fig. 49(k).

Referring to Figs. 50 to 57 (c) there is illustrated a hand access device according to the invention for use in hand assisted laparoscopic surgery comprising a first ring element 200, a second ring element 201 and a sleeve 202 of pliable material with a first end mounted to the first ring element 200 and a second end mounted to the second ring element 200. For ease of reference the ring elements 200, 201 have associated location markings 205, 206 respectively. The sleeve 202 is twisted and has a normally closed access opening 207 and the sleeve is movable on insertion of a surgeon's hand/arm 210 through the access opening 207.

A biasing means is provided to bias the sleeve 202 to close the access opening 207. The biasing may be provided by the properties of the sleeve which may be pretensioned, or by using a separate spring element. In this case the spring element 215 is a strip of elastic material 215 which is mounted at one end to the first ring 201. The elastic strip 215 causes the rings to be biased into a rest position at which the opening 207 is closed. On insertion of a surgeon's hand the entry force acts against the biasing of the elastic strip 215 and the rings 200, 201 rotate relative to one another as evidenced by the locator marks 205, 206.

In the invention, the sealing member has a normally closed access opening. The access opening is only sufficient to allow a surgeon to insert his hand and forearm through the sleeve whilst maintaining substantial continuous sealing engagement between the sleeve and the surgeon's hand/forearm, thus ensuring that there is substantially no gas leakage and maintaining pneumoperitoneum. The device is very easily manufactured and, most importantly, is extremely easy for a surgeon to use, as a sealed access port is provided through which a surgeon can easily insert his arm and forearm. It will be noted that the biasing ensures that the access opening substantially exactly matches the contours of the inserted hand/forearm and automatically opens and closes as required.

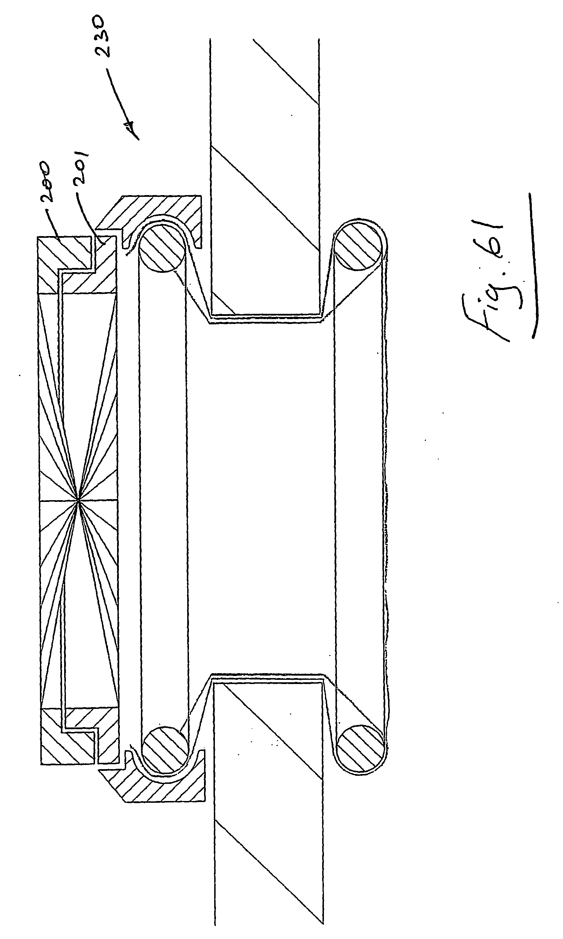

In another embodiment; as illustrated in Figs. 58 (a) to 60 (c), the spring element may be a coiled spring 220 which normally biases the rings 200, 201 and hence the sealing member 202 in such a way as to close the opening.

Referring to Fig. 61 the hand access device of Figs 50 to 60 is shown mounted to a retractor 230 such as a retractor as described above.

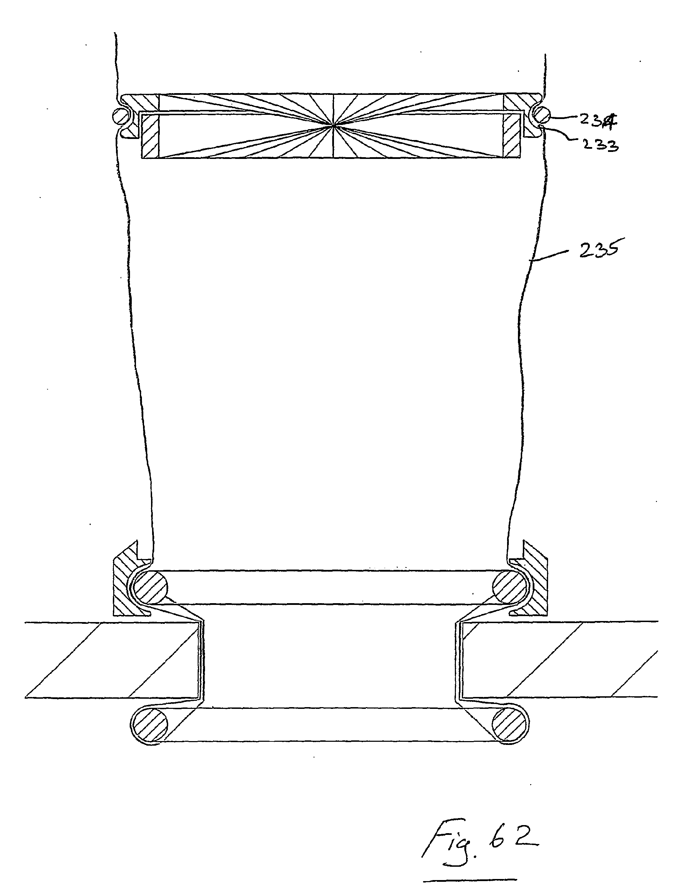

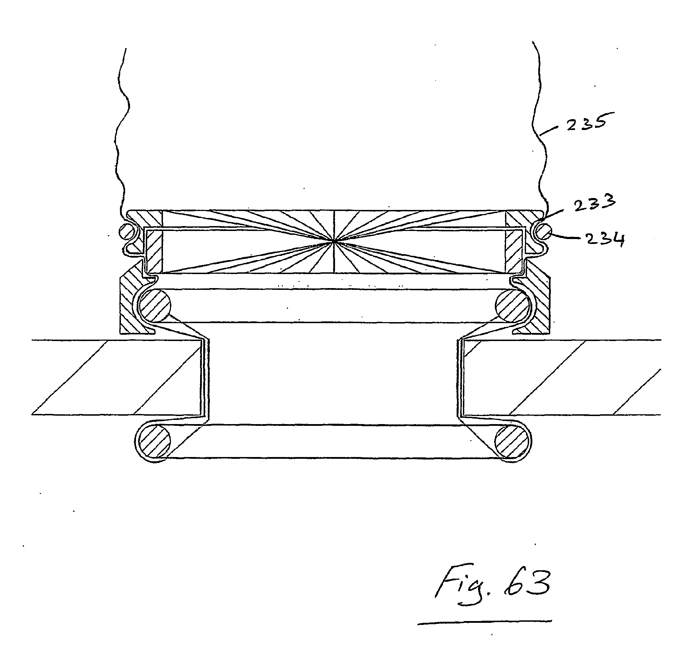

Referring to Figs 62 and 63 the hand access device is shown being mounted to another type of retractor 240. In this case the first ring element 200 has a circumferentially extending groove 233 and an associated ring 234 with a retractor sleeve section 235 accommodated therebetween to permit sliding action of the hand access device relative to the retractor sleeve section 235.

It will be appreciated that the hand access devices of the invention can be used with any suitable retractor or other similar device.

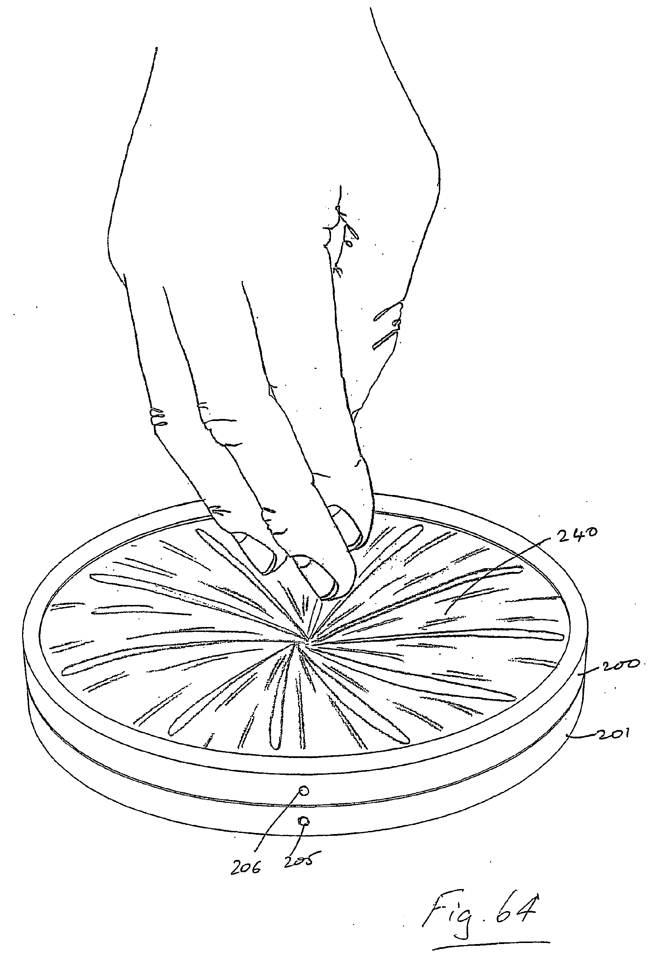

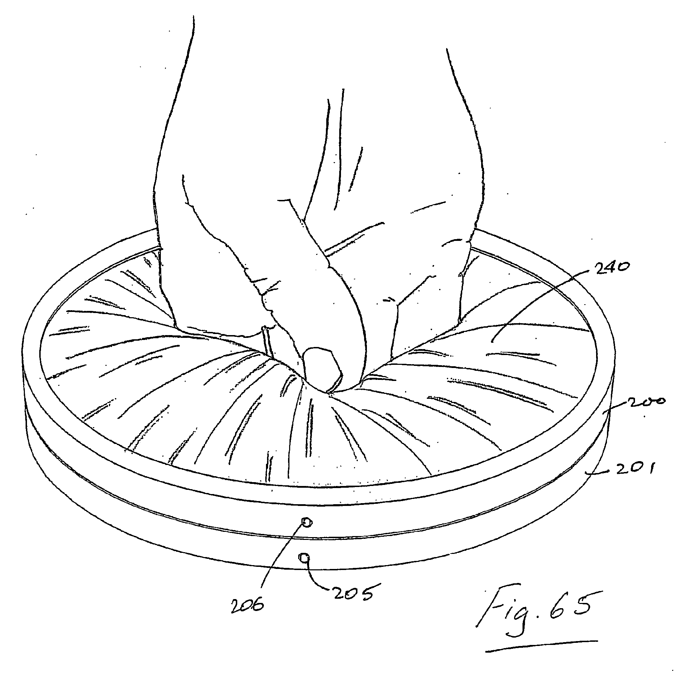

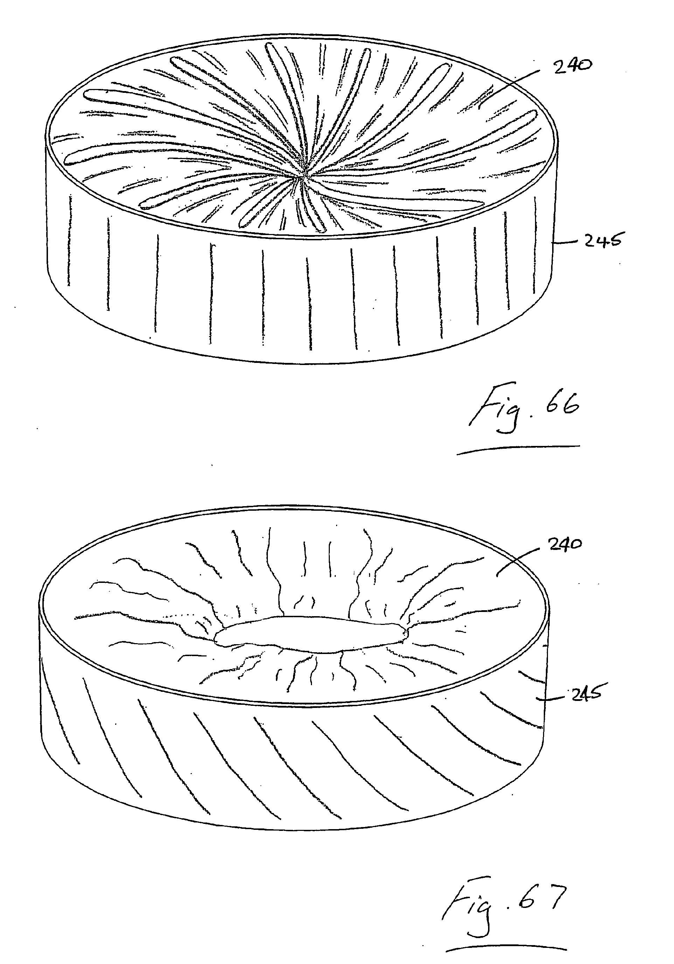

Referring to Figs. 64 and 65 there is illustrated another hand access device which is similar to the device of Figs. 50 to 57 except that in this case the biasing to close the access port is provided by the properties of the sleeve 240 which in this case is pretensioned. The surgeon, on insertion of his hand/arm acts to overcome the tension in the sleeve 240 sufficient to allow hand insertion whilst still maintaining sealing engagement to the surgeon's hand/arm. This configuration will also be apparent from Figs 66 and 67. The twisted sealing member such as a sleeve 240 defining an iris is shown in Fig. 66 with a strong outer resilient material 245. As a surgeon inserts his hand the twist in the sleeve is transferred to the outer resilient material 245 with the applied force. In this and in other configurations described above the sealing member may be a suitable elastomeric material. In Fig. 67 the hand is removed for clarity, in reality on removal of the hand the system will revert to the closed configuration of Fig. 66.

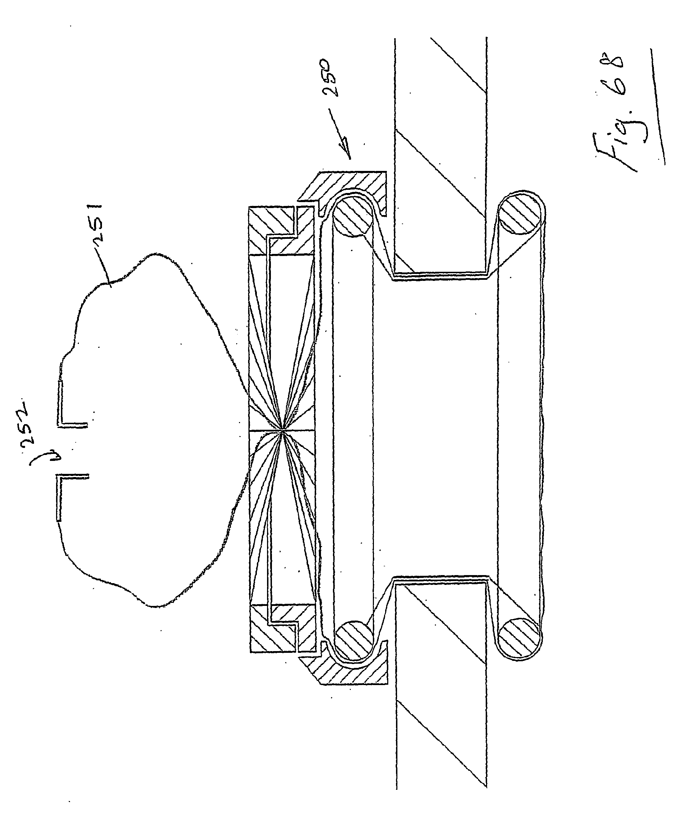

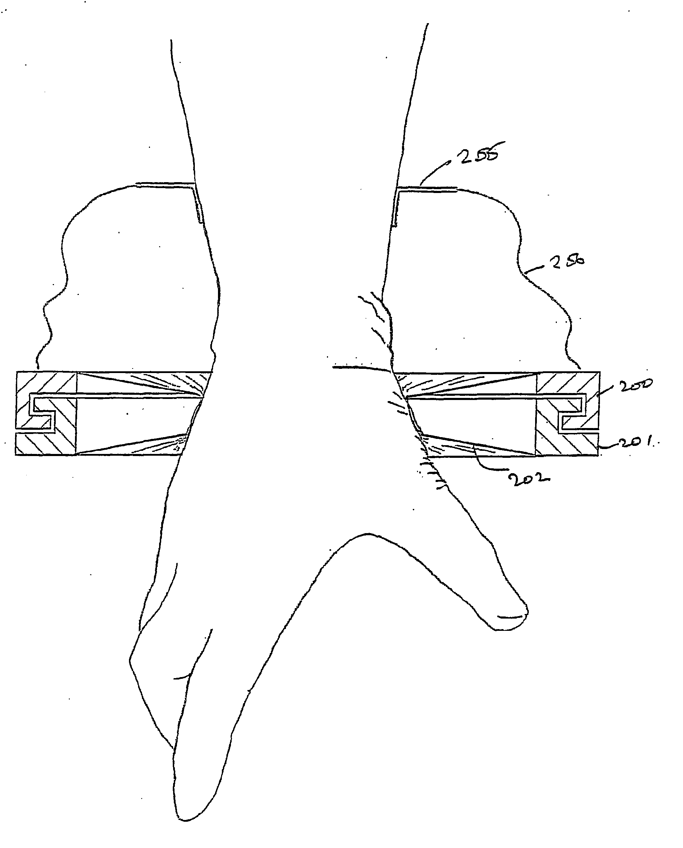

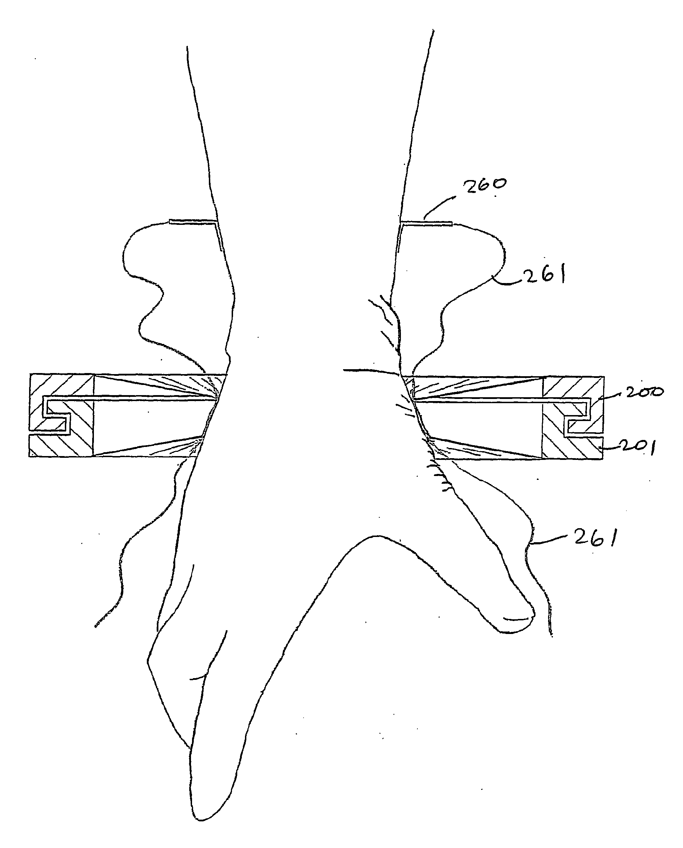

Referring now to Fig. 68 there is illustrated an assembly of a hand access device of the invention with a retractor 250 having an excess retractor sleeve section 251 provided with an outer lip seal 252 for sealing engagement to the arm of a surgeon. The excess retractor sleeve section may be used to externalise an organ during a surgical procedure. In Fig 69 a lip seal 255 is provided in a sleeve section 250 mounted to the ring element 200. In Fig. 70 a lip seal 260 is provided on a separate sleeve section 261.

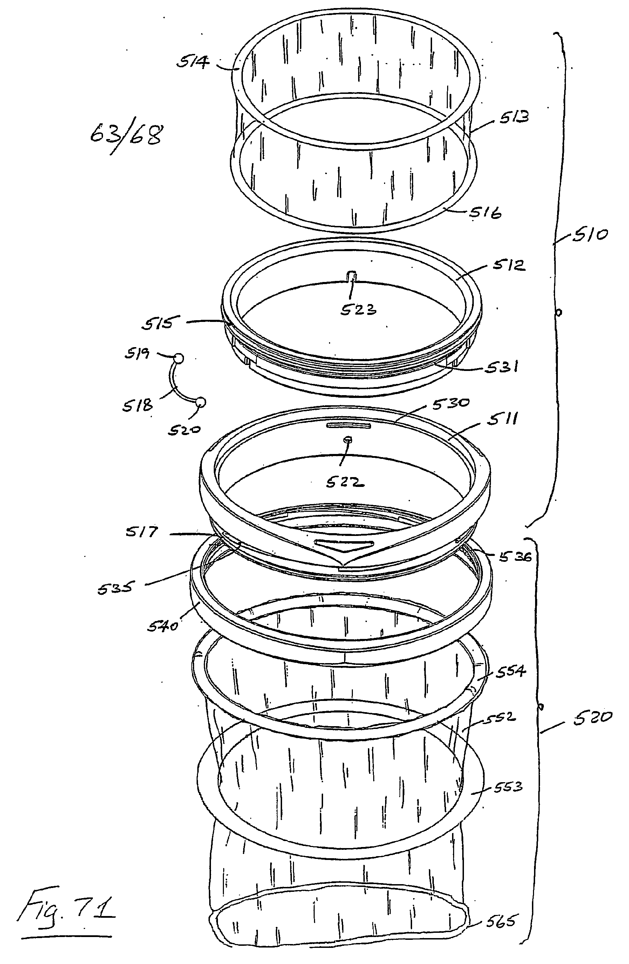

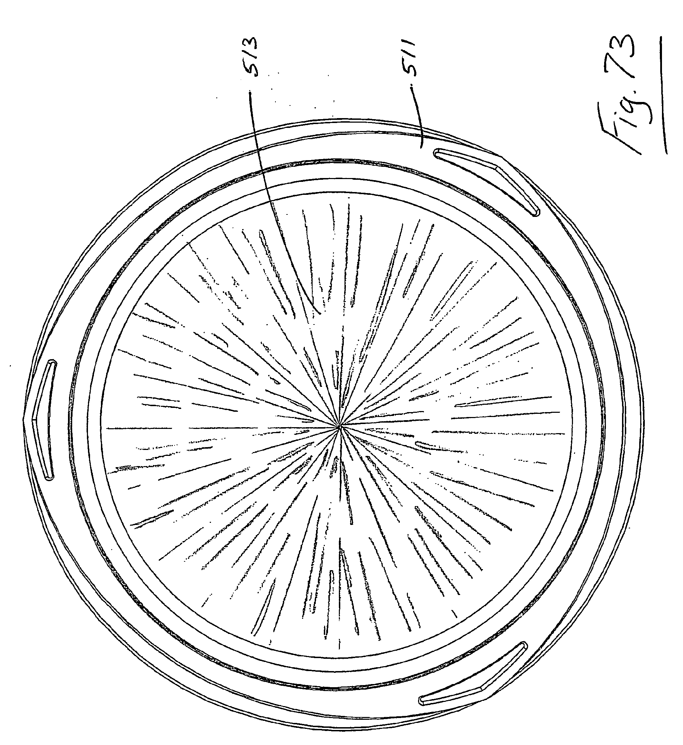

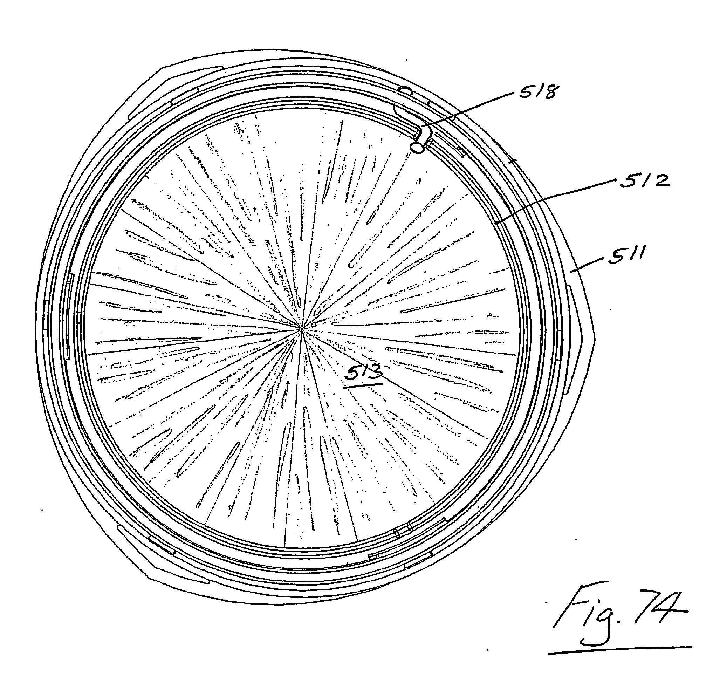

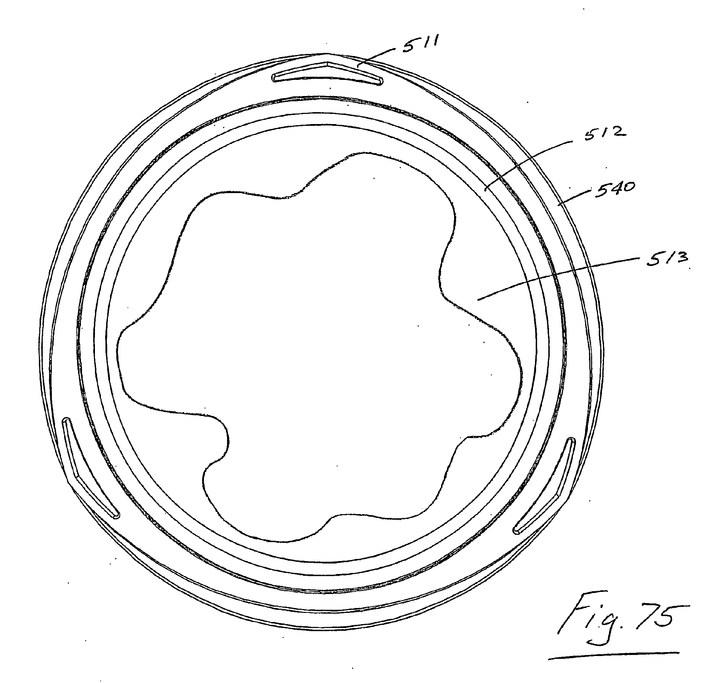

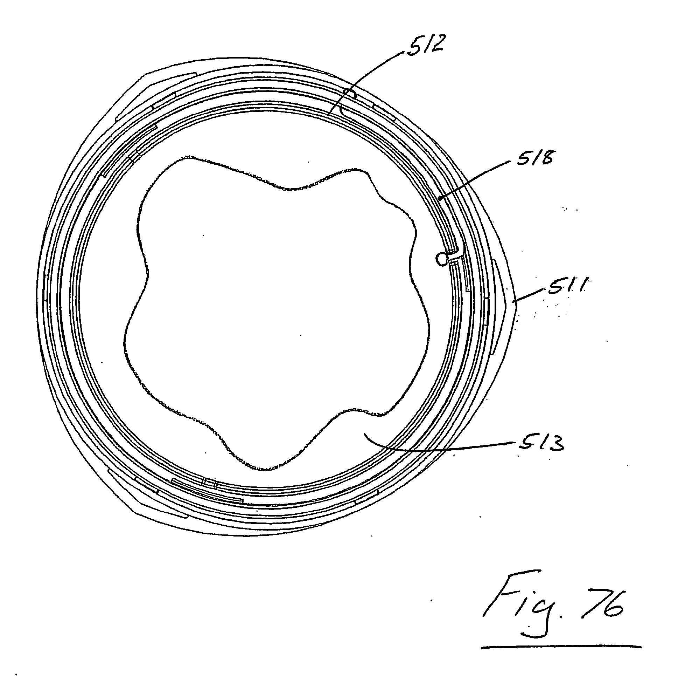

Referring to Figs. 71 to 76 there is illustrated an assembly 500 of the invention which comprises a retractor 501 and a normally closed iris valve 502 releasably mounted to the retractor 501. The retractor 501 is similar to the retractors described above such as with reference to Figs. 1 to 10. The iris valve 502 is similar to the iris valves described above such as with reference to Figs. 50 to 57(c).

The iris comprises the components within the chain bracket 510 in Fig. 71 and the retractor comprises the components within the chain bracket 520 in Fig. 71.

The iris 502 comprises a fixed outer iris ring member 511 and an inner rotatable ring member 512. The inner ring member 512 is in this case a snap fit and is free to rotate relative to the outer ring member 511. The snap fit engagement is through an annular rib 530 on the outer ring member 511 and a corresponding annular groove 531 in the inner ring member 512. A flexible iris-forming sleeve 513 extends between the inner and outer ring members 511, 512. The sleeve 513 has a first elasticated ring or band 514 at one end for anchoring in a corresponding engagement channel 515 in the inner ring member 512 and a second elasticated band 514 at the other end for anchoring in a corresponding engagement channel 517 in the outer ring member 511. Thus, one end of the iris-forming sleeve 513 is anchored to the movable ring member 512 and the other end is anchored to the fixed ring member 512 so that rotation of the ring member 512 relative to the fixed ring member 511 will result in twisting or untwisting of the sleeve, forming an iris valve.

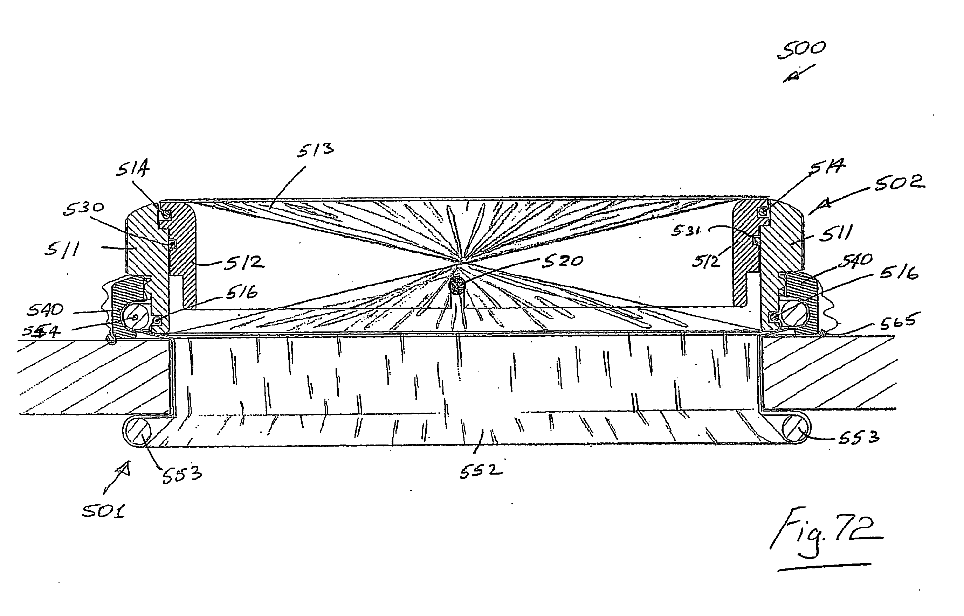

The iris valve 502 is biased into a normally closed position (Figs. 72 to 74) by a spring which in this case is in the form of a strip of elastic material 518 having enlarged head portions 519, 521 at the ends thereof for location and engagement of one end of the spring 518 in a spring locating hole 522 in the fixed ring member 511 and for location and engagement of the other end of the spring 518 in a spring locating slot 523 in the rotatable ring member 512. The spring 518 biases the iris-forming sleeve 513 into the normally closed position. On insertion of an object such as a surgeons hand, the biasing force of the spring 518 is counteracted causing partial opening of the iris valve whilst still remaining sealing engagement of the iris sleeve with the object passing therethrough. A twisting action of the object as it is being inserted will aid overcoming of the spring biasing action, in some cases. The operation of the iris is described in more detail above.

The iris forming sleeve 513 has a length in the unassembled untwisted configuration of Fig. 71 that is preferably less than or equal to the diameter of the sleeve 513. We have found that this is advantageous in optimising the operation of the iris by ensuring full closure of the iris whilst ensuring that excess sleeve material, on twisting, is minimised.

The iris valve 502 is in this case releasably mounted to the retractor 501, Thus, the iris 502 may be used independently of the retractor 501 and vice versa. In this instance the iris valve is screw threadingly engagable with the retractor, the outer ring 511 of the iris having a thread 535 for connection to the retractor 501. The retractor 501 in turn has tabs 536 which project inwardly from a retractor top ring 540 for engagement with the screw thread 535 of the outer ring 511. Any suitable interconnection may be provided.

The retractor 501 comprise a sleeve 552, a distal member provided by a distal ring 553. of resilient material such as an 0-ring and a proximal member provided by a proximal ring 554 which may also be an 0-ring.

The sleeve 552 is of any suitable material such as of pliable plastics film material and comprises a distal portion 555 for insertion through an incision 556, in this case made in a patient's abdomen 557, and a proximal portion 558 for extending from the incision 556 and outside of the patient.

In this case the distal ring 553 is not fixed to the sleeve 552 but rather the sleeve is led around the ring 553 and is free to move axially relative to the distal ring 553 somewhat in the manner of a pulley. The proximal ring 554 is fixed to the sleeve 552, in this case at the proximal inner end thereof. The sleeve 552 terminates in a handle or gripping portion which in this case is reinforced by a gripping ring 565.

As described above with reference to Figs. 1 to 10, to configure the retractor device a sleeve 552 is first provided with the gripping ring 565 fixed at one end and the proximal ring 554 fixed at the other end [Figs. 3, 5]. The distal ring 553 is then placed over the sleeve 552. The gripping ring 565 is then used to manipulate the sleeve 552 so that the sleeve 552 is folded back on itself into the configuration of Figs. 1 and 2 in which the gripping ring 565 is uppermost. The sleeve extends from the proximal ring 554 and the distal ring 553 is contained between inner and outer layers of the sleeve 2. The device is now ready for use.

The resilient distal ring 553 is scrunched up and inserted through the incision 556 with the distal end 555 of the sleeve 552 as illustrated in Fig. 4. The sleeve 552 is then pulled upwardly in the direction of the arrows A in Figs. 8 to 10. On pulling of the sleeve 552 upwardly the sleeve outer layer is pulled up while the sleeve inner layer is drawn around the proximal ring 553. This results in shortening the axial extent between the proximal ring 554 and the distal ring 553, tensioning the sleeve 552 and applying a retraction force to the margins of the incision 556. The system appears to be self locking because we have observed that when tension is applied to the sleeve 552 and the pulling force is released the rings 553, and 554 remain in position with a retraction force applied. Frictional engagement between the layers of the sleeve in this configuration may contribute to this self locking. As the incision is being retracted the margins are also protected by the sleeve. On retraction, an access port is provided, for example for a surgeon to insert his hand and/or an instrument to perform a procedure.

In this instance the sleeve gripping ring 565 is led over the retractor top ring 540 and the gripping ring 565 is retained outside of the top ring 540 as illustrated in Fig. 72. The retractor top ring 540 provides a guide member for the retractor proximal ring 554. The guide member or top ring 540 is in the form of an annular ring member with an inwardly facing C-shaped groove which is sized to accommodate the ring 554 as illustrated. The outer layer of the sleeve 552 is interposed between the ring 554 and the guide 540 to further control the pulling of the sleeve and thereby further controlling the application of the retraction force. The guide 540 also assist in stabilising the proximal ring 554.

The invention is not limited to the embodiments hereinbefore described, with reference to the accompanying drawings, which may be varied in construction and detail.

## Claims

1. A surgical device (150) for use in surgery comprising:-
a mounting element;
a sealing member (202) of pliable material mounted to the mounting element, the sealing member (202) having a normally closed access opening (207); and
at least a portion of the sealing member (202) being movable on insertion of an object such as a surgeon's hand (210) to open the access opening (207) whilst maintaining substantial sealing engagement with the object; and
a spring element (215) to bias the sealing member (202) to close the access opening (207).

2. A device as claimed in claim 1 wherein the mounting element comprises a first ring element (200) and a second ring element (201) and the sealing member (202) extends between the ring elements (200, 201).

3. A device as claimed in claim 1 or 2 wherein the sealing member (202) comprises a sleeve.

4. A device as claimed in any of claims 1 to 3 wherein the sealing member (202) is of an elastomeric material.

5. A device as claimed in any of claims 2 to 4 wherein one ring element (200) is located within the other ring element (201).

6. A device as claimed in any of claims 2 to 5 wherein the sealing member (202) is a sleeve, one end of the sleeve (202) being mounted to one ring element (200) and an opposite end of the sleeve (202) being mounted to the other ring element (201).

7. A device as claimed in any of claims 2 to 6 wherein the mounting ring elements (200, 201) have locators (205, 206) for the spring element extending between the ring elements (200, 201).

8. A device as claimed in any of claims 2 to 7 wherein the sealing member (202) comprises a sleeve having an untwisted configuration and a twisted sealing configuration, the sleeve (202) in the untwisted configuration having a length which is less than a diameter of the sleeve (202).

9. A device as claimed in any of claims 2 to 8 wherein one ring element (200) is rotatable relative to the other ring element (201).

10. A device as claimed in any of claims I to 9 wherein the mounting element has an attachment for mounting to another device such as a retractor (501).

11. A device as claimed in claim 10 wherein the attachment comprises a groove.

12. A device as claimed in claim 10 or 11 wherein the other device comprises a sleeve element (552) and the attachment is engagable with the sleeve element (552).

13. A device as claimed in any of claims 10 to 12 wherein the mounting element is movable relative to the other device,

14. A device as claimed in any of claims 10 to 13 wherein the other device comprises a sealing device (161).

15. A hand access device for use in hand assisted laparoscopic surgery comprising a device (150) as claimed in any of claims 1 to 14.

16. A surgical device assembly comprising a retractor (501) and a device (150) as claimed in any of claims 1 to 15.

17. An assembly as claimed in claim 16 wherein the device (150) is releasably mountable to the retractor (501).

## Patentansprüche

1. Chirurgische Vorrichtung (150) zum Gebrauch bei Chirurgie, umfassend:
ein Befestigungselement;
ein Abdichtungselement (202) aus biegsamem Material, das an dem Befestigungselement angebracht wird, wobei das Abdichtungselement (202) eine normalerweise geschlossene Zugangsöffnung (207) aufweist; und
wobei mindestens ein Teil des Abdichtungselements (202) bei Einführung eines Objekts wie zum Beispiel der Hand eines Chirurgen (210) zum Öffnen der Zugangsöffnung (207) bewegbar ist, während derselbe im Wesentlichen in abdichtendem Eingriff mit dem Objekt bleibt; und
ein Federelement (215) zum Vorspannen des Abdichtungselements (202), um die Zugangsöffnung (207) zu verschließen.

2. Vorrichtung nach Anspruch 1, bei der das Befestigungselement ein erstes Ringelement (200) und ein zweites Ringelement (201) aufweist, und das Abdichtungselement (202) sich zwischen den Ringelementen (200, 201) erstreckt.

3. Vorrichtung nach Anspruch 1 oder 2, bei der das Abdichtungselement (202) eine Hülse aufweist.

4. Vorrichtung nach einem der Ansprüche 1 bis 3, bei der das Abdichtungselement (202) aus einem elastomeren Material besteht.

5. Vorrichtung nach einem der Ansprüche 2 bis 4, bei der ein Ringelement (200) sich innerhalb des anderen Ringelements (201) befindet.

6. Vorrichtung nach einem der Ansprüche 2 bis 5, bei der das Abdichtungselement (202) eine Hülse ist, wobei ein Ende der Hülse (202) an einem Ringelement (200) angebracht wird und ein entgegengesetztes Ende der Hülse (202) an dem anderen Ringelement (201) angebracht wird.

7. Vorrichtung nach einem der Ansprüche 2 bis 6, bei der die Befestigungsringelemente (200, 201) Lokalisierer (205, 206) für das Federelement aufweisen, das sich zwischen den Ringelementen (200, 201) erstreckt.

8. Vorrichtung nach einem der Ansprüche 2 bis 7, bei der das Abdichtungselement (202) eine Hülse mit einer nicht verdrehten Konfiguration und einer verdrehten Abdichtungskonfiguration aufweist, wobei die Hülse (202) in der unverdrehten Konfiguration eine Länge aufweist, die kleiner als ein Durchmesser der Hülse (202) ist.

9. Vorrichtung nach einem der Ansprüche 2 bis 8, bei der ein Ringelement (200) in Bezug zu dem anderen Ringelement (201) drehbar ist.

10. Vorrichtung nach einem der Ansprüche 1 bis 9, bei der das Befestigungselement eine Halterung zum Anbringen an einer anderen Einrichtung wie zum Beispiel einem Retraktor (501) aufweist.

11. Vorrichtung nach Anspruch 10, bei der die Halterung eine Nut aufweist.

12. Vorrichtung nach Anspruch 10 oder 11, bei der die andere Einrichtung ein Hülsenelement (552) aufweist und die Halterung mit dem Hülselement (552) in Eingriff gebracht werden kann.

13. Vorrichtung nach einem der Ansprüche 10 bis 12, bei der das Befestigungselement in Bezug zu der anderen Einrichtung beweglich ist.

14. Vorrichtung nach einem der Ansprüche 10 bis 13, bei der die andere Einrichtung eine Abdichtungseinrichtung (161) aufweist.

15. Handzugangsvorrichtung zum Gebrauch bei handunterstützter laparoskopischer Chirurgie, die eine Vorrichtung (150) nach einem der Ansprüche 1 bis 14 aufweist.

16. Chirurgische Vorrichtungsbaugruppe, die einen Retraktor (501) und eine Einrichtung (150) nach einem der Ansprüche 1 bis 15 aufweist.

17. Baugruppe nach Anspruch 16, bei der die Einrichtung (150) lösbar an dem Retraktor (501) angebracht werden kann.

## Revendications

1. Dispositif chirurgical (150) à des fins d'utilisation en chirurgie comportant :
un élément de montage ;
un organe d'étanchéité (202) en matière souple monté sur l'élément de montage, l'organe d'étanchéité (202) ayant une ouverture d'accès normalement fermée (207) ; et
au moins une portion de l'organe d'étanchéité (202) étant mobile lors de l'insertion d'un objet tel que la main d'un chirurgien (210) pour ouvrir l'ouverture d'accès (207) tout en maintenant un important engagement étanche avec l'objet ; et
un élément à ressort (215) destiné à solliciter l'organe d'étanchéité (202) à fermer l'ouverture d'accès (207).

2. Dispositif selon la revendication 1, dans lequel l'élément de montage comporte un premier élément de type anneau (200) et un deuxième élément de type anneau (201) et l'organe d'étanchéité (202) se prolonge entre les éléments de type anneau (200, 201).

3. Dispositif selon la revendication 1 ou la revendication 2, dans lequel l'organe d'étanchéité (202) comporte un manchon.

4. Dispositif selon l'une quelconque des revendications 1 à 3, dans lequel l'organe d'étanchéité (202) est en matière élastomère.

5. Dispositif selon l'une quelconque des revendications 2 à 4, dans lequel un élément de type anneau (200) est situé à l'intérieur de l'autre élément de type anneau (201).

6. Dispositif selon l'une quelconque des revendications 2 à 5, dans lequel l'organe d'étanchéité (202) est un manchon, une extrémité du manchon (202) étant montée sur un élément de type anneau (200) et une extrémité opposée du manchon (202) étant montée sur l'autre élément de type anneau (201).

7. Dispositif selon l'une quelconque des revendications 2 à 6, dans lequel les éléments de type anneau de montage (200, 201) ont des pièces de repérage (205, 206) pour l'élément à ressort se prolongeant entre les éléments de type anneau (200,201).

8. Dispositif selon l'une quelconque des revendications 2 à 7, dans lequel l'organe d'étanchéité (202) comporte un manchon ayant une configuration détordue et une configuration d'étanchéité tordue, le manchon (202) dans la configuration détordue ayant une longueur qui est inférieure au diamètre du manchon (202).

9. Dispositif selon l'une quelconque des revendications 2 à 8, dans lequel un élément de type anneau (200) est rotatif par rapport à l'autre élément de type anneau (201).

10. Dispositif selon l'une quelconque des revendications 1 à 9, dans lequel l'élément de montage a un accessoire à des fins de montage sur un autre dispositif tel qu'un écarteur (501).

11. Dispositif selon la revendication 10, dans lequel l'accessoire comporte une rainure.

12. Dispositif selon la revendication 10 ou la revendication 11, dans lequel l'autre dispositif comporte un élément à manchon (552) et l'accessoire est en mesure de s'engager avec l'élément à manchon (552).

13. Dispositif selon l'une quelconque des revendications 10 à 12, dans lequel l'élément de montage est mobile par rapport à l'autre dispositif.

14. Dispositif selon l'une quelconque des revendications 10 à 13, dans lequel l'autre dispositif comporte un dispositif d'étanchéité (161).

15. Dispositif d'accès de la main à des fins d'utilisation en chirurgie laparoscopique assistée par la main comportant un dispositif (150) selon l'une quelconque des revendications 1 à 14.

16. Ensemble de dispositif chirurgical comportant un écarteur (501) et un dispositif (150) selon l'une quelconque des revendications 1 à 15.

17. Ensemble selon la revendication 16, dans lequel le dispositif (150) est en mesure d'être monté de manière libérable sur l'écarteur (501).
